# EUROPEAN PATENT APPLICATION

(11) **EP 4 092 125 A1**
(43) Date of publication of application: **23.11.2022**
(21) Application number: 21174141.8
(22) Date of filing: 17.05.2021
(51) Int. Cl.: C12N 15/85, C07K 1/00, C07K 14/47

(54) **METHOD FOR PRODUCING A BIOMOLECULE BY A CELL**

(71) Applicant: Hochschule Biberach, 88400 Biberach (DE)
(72) Inventor: OTTE, Kerstin, 89073 Ulm (DE); HANDRICK, René, 88400 Biberach (DE); ZEH, Nikolas, 88451 Dettingen (DE)
(74) Representative: PATERIS Patentanwälte PartmbB

(57) **Abstract**

Disclosed is a method for producing a biomolecule by a cell cultured *in vitro,* the method comprising the steps of: (a) propagating the cell in a cell culture, wherein the cell is transformed with a vector, wherein the vector comprises (i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences; (ii) a promoter that is active in the cell, wherein the promoter is operably linked to the HRE; (iii) a nucleic acid sequence encoding the biomolecule, wherein the nucleic acid sequence is operably linked to the promoter; and (b) isolating the biomolecule from the cell culture. Further disclosed is a cell for producing a biomolecule in *vitro.*

## Description

### Field of the invention

The present invention relates to a method for producing a biomolecule by a cell cultured *in vitro.* The present invention further relates to a cell for producing a biomolecule *in vitro.*

### Background of the invention

A large number of biopharmaceuticals such as antibodies, cytokines and hormones is produced using biotechnological methods. To do so, a given biopharmaceutical is expressed in suitable cells that are cultured in large scale cell cultures and the biopharmaceutical is then isolated from the cell culture. Suitable cells can be lower organisms such as bacteria and yeast or cells of cell lines derived from higher order organisms such as mammals, depending on the type of biopharmaceutical to be produced.

To increase the efficiency of the production of biopharmaceuticals and other biomolecules while ensuring maximal product quality, cells, cell lines and production processes are continuously optimized. The optimization of cell lines includes, for example, optimizing cell morphology and metabolism, improving growth, increasing viability, reducing apoptosis, senescence and autophagy, increasing productivity as well as introducing specific changes to post-translational protein modifications. To optimize cell lines, various cell line engineering approaches that are generally time- and cost-intensive such as gene knockout of unfavourable genes or gene clusters are applied. Another commonly applied technique is the transformation of the cells with a vector. In this way, the genome of the cells does not need to be modified. The optimization of such vectors is an area of intense research.

The optimization of production processes includes, for example, increasing gassing rate and stirring speed as well as upscaling cell culture volume. To optimize production processes, to date, continuous processes such as perfusion processes are increasingly used. Continuous processes allow higher cell densities compared to non-continuous processes and can thus be performed in smaller cell culture volumes as well as in shorter periods of time. They also provide further advantages such as higher product quality, higher product yield and/or cost savings. However, continuous processes also have certain limitations, in particular oxygen deficiency at high cell densities. Oxygen deficiency may negatively affect cellular parameters, cell productivity and/or product quality.

Therefore, new methods and tools for producing biomolecules are needed that overcome the current limitations, in particular that increase the efficiency of biomolecule production in high cell density perfusion processes.

### Summary of the invention

In a first aspect, the present invention relates to a method for producing a biomolecule by a cell cultured *in vitro,* the method comprising the steps of:
(a) propagating the cell in a cell culture, wherein the cell is transformed with a vector, wherein the vector comprises
   (i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
   (ii) a promoter that is active in the cell, wherein the promoter is operably linked to the HRE;
   (iii) a nucleic acid sequence encoding the biomolecule, wherein the nucleic acid sequence is operably linked to the promoter;
   and
(b) isolating the biomolecule from the cell culture.

In a second aspect, the present invention relates to a cell for producing a biomolecule *in vitro,* wherein the cell is transformed with a vector, wherein the vector comprises
(i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
(ii) a promoter that is active in the cell, wherein the promoter is operably linked to the HRE;
(iii) a nucleic acid sequence encoding the biomolecule, wherein the nucleic acid sequence is operably linked to the promoter.

### Brief description of the figures

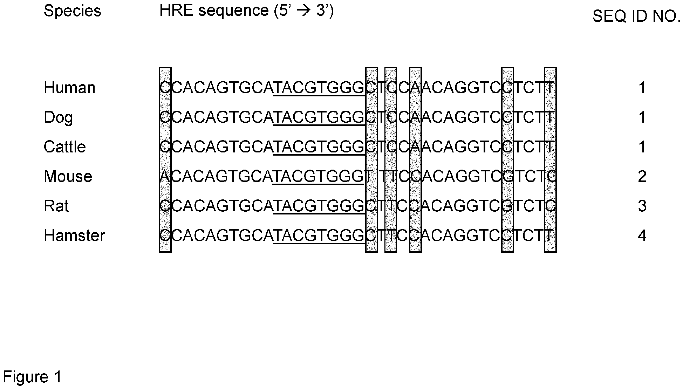

Fig. 1 shows the sequences of the hypoxia-responsive element (HRE) of the VEGF-A gene of various species. The conserved binding site of the hypoxia-inducible factor (HIF) is underlined. Sequence alterations between some of the species occur at a few specific positions in the HRE sequence as marked by vertical boxes.

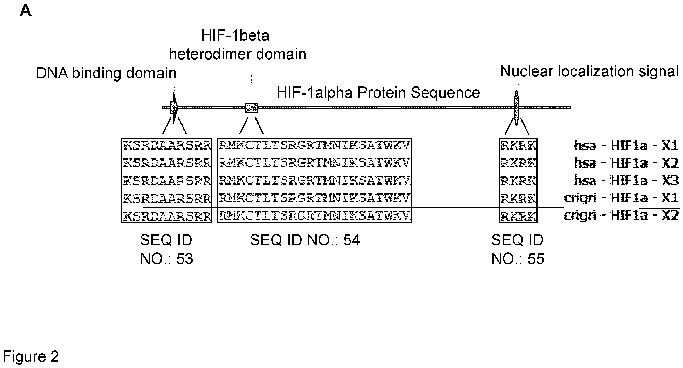

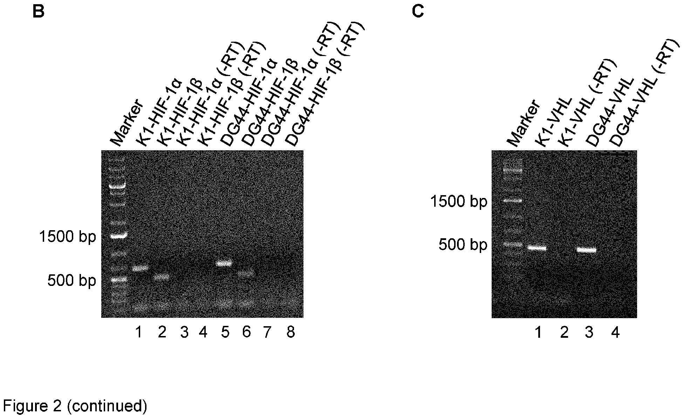

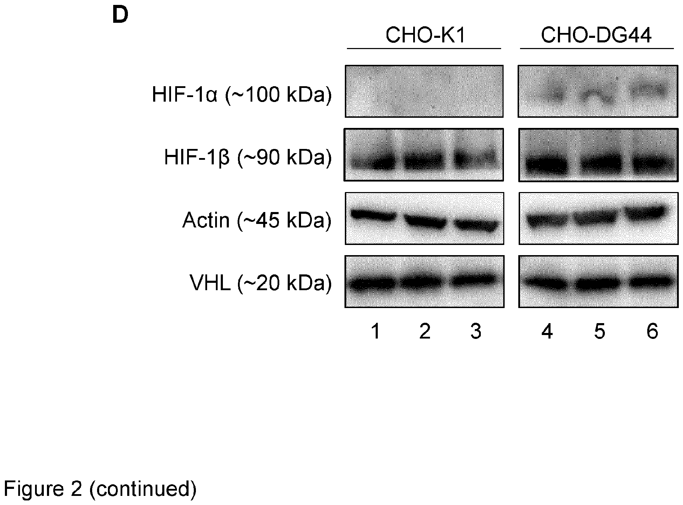

Fig. 2 shows the molecular biological identification of crucial factors for the detection of hypoxia by Chinese hamster ovary (CHO) cells. (A) Alignment of the amino acid sequences of hypoxia-inducible factor-1α (HIF-1α) protein isoforms from human (hsa) and hamster (crigri) with highlighted functional domains. (B) PCR on cDNA detecting HIF-1α (Lane 1+5) and HIP-1β (Lane 2+6) in CHO-K1 (Lane 1-4) and CHO-DG44 (Lane 5-8). Lane 3-4 and 7-8 represent negative controls lacking reverse transcriptase (-RT). (C) PCR on cDNA detecting Von Hippel-Lindau protein (VHL) (Lane 1+3) in CHO-K1 (Lane 1-2) and CHO-DG44 (Lane 3-4). Lane 2+4 represent the negative -RT control. (D) Western blot on HIF-1a (top panel), HIP-1β (second top panel) and VHL (bottom panel) in CHO-K1 (Lane 1-3) and CHO-DG44 (Lane 4-6) using 30 µg of protein per lane.

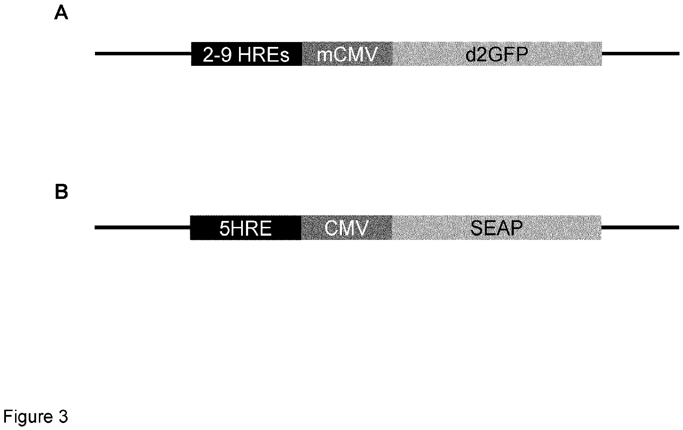

Fig. 3 shows a schematic view of fragments of the vector constructs generated by the inventors. (A) Fragment of the vector constructs used for validation studies in CHO cells. 2-9 HREs denotes 2 to 9 copies of the HRE sequence of the human VEGF-A gene or the human EPO gene, mCMV denotes the minimal CMV promoter and d2GFP denotes the nucleic acid sequence encoding the biomolecule. In this example, the biomolecule is d2GFP, which is a destabilized variant of the green fluorescent protein (GFP). (B) Fragment of the vector construct used for recombinant protein expression studies in CHO cells. 5HRE denotes five copies of the HRE sequence of the human VEGF-A gene, CMV denotes the complete CMV promoter and SEAP denotes the nucleic acid sequence encoding the biomolecule. In this example, the biomolecule is SEAP (secreted embryonic alkaline phosphatase).

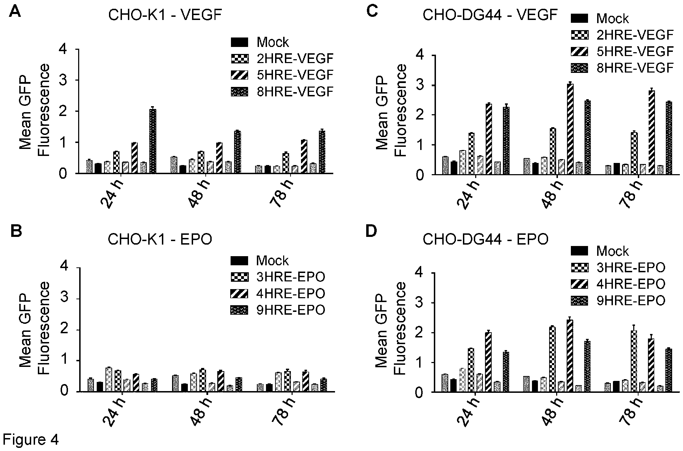

Fig. 4 shows the identification of the most promising vector constructs under undefined hypoxic conditions. (A+B) Flow cytometry analysis of static or shaken cultivated CHO-K1 pools, stably expressing destabilized GFP (d2GFP) under the control HREs from VEGF-A (A) or EPO (B) origin. Grey bars stand for shaken, while black bars represent static cultured pools. (C+D) Flow cytometry analysis of static or shaken cultivated CHO-DG44 pools, stably expressing destabilized GFP (d2GFP) under the control of HREs from VEGF (C) or EPO (D) origin. Grey bars stand for shaken, while black bars represent static cultured pools [n = 3 replicates; Mean ± standard deviation (SD)].

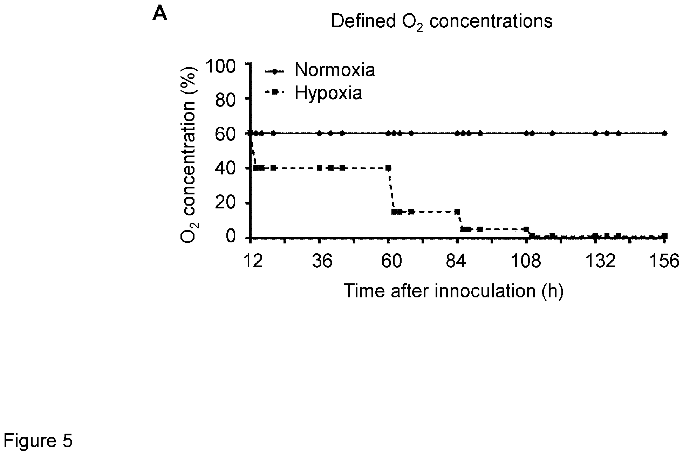

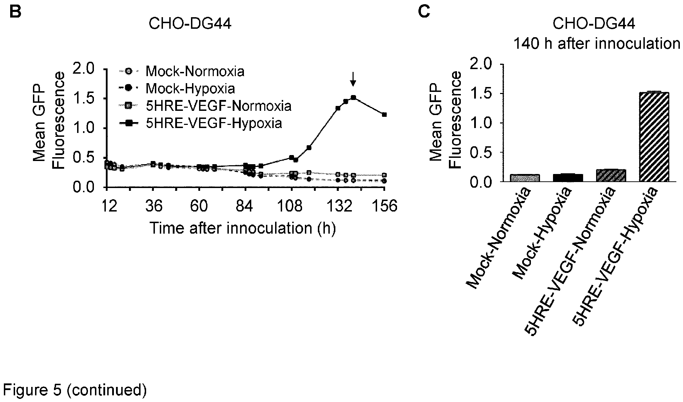

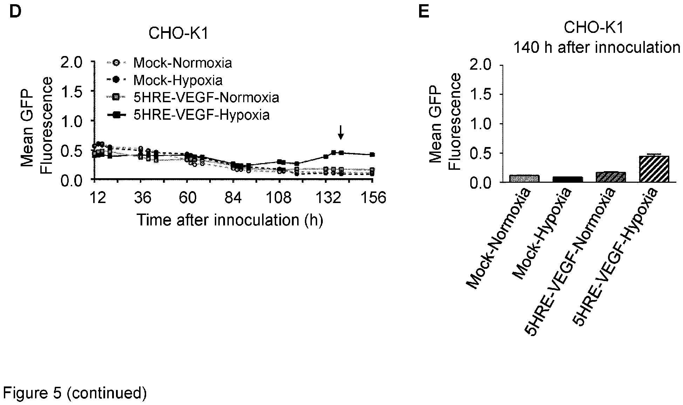

Fig. 5 shows the characterization of the response of CHO-DG44 and CHO-K1 cells to defined O₂-concentrations. (A) Progression of the controlled oxygen concentration during fermentation in a normoxic batch (solid line) and a stepwise hypoxic cultivation (dotted line). (B) Mean fluorescence of CHO-DG44-Mock (dotted lines) and CHO-DG44-5HRE-VEGF (solid lines) cells cultured in a batch fermentation over 156 h under normoxic and stepwise hypoxic conditions. (C) Mean fluorescence of normoxic and hypoxic cultured CHO-DG44-Mock (non-striped bars) and CHO-DG44-5HRE-VEGF (striped bars) cells after 140 h. (D) Mean fluorescence of CHO-K1-Mock (dotted lines) and CHO-K1-5HRE-VEGF (solid lines) cells cultured in a batch fermentation over 156 h under normoxic and stepwise hypoxic conditions. (E) Mean fluorescence of normoxic and hypoxic cultured CHO-K1-Mock (non-striped bars) and CHO-K1-5HRE-VEGF (striped bars) cells after 140 h. Statistical analysis was conducted using one-way ANOVA with Bonferroni correction or students t-test (F) [n = 3 replicates; Mean ± SD].

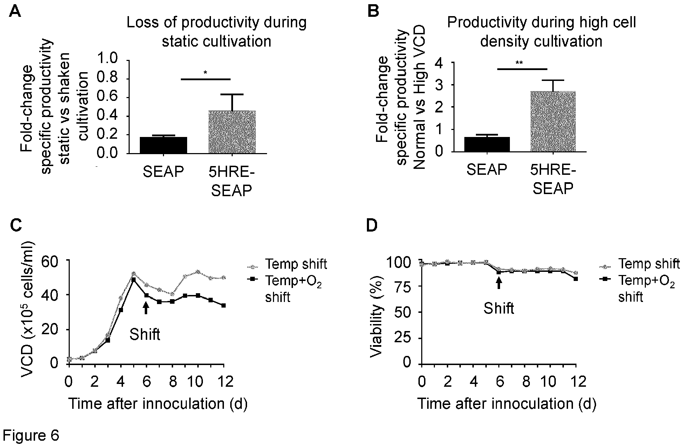

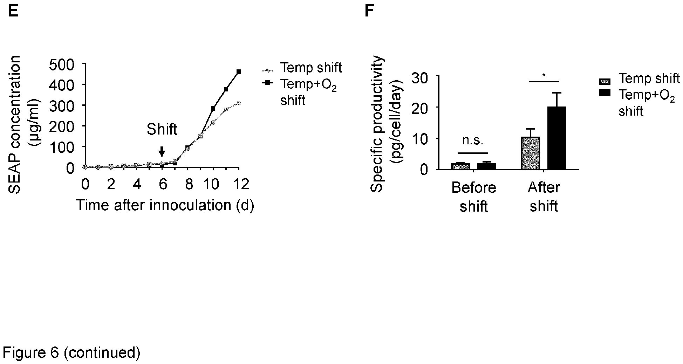

Fig. 6 shows inducible secreted embryonic alkaline phosphatase (SEAP) expression exploiting hypoxia during oxygen deprivation conditions. (A) Specific productivity of CHO-DG44-SEAP and CHO-DG44-5HRE-SEAP cells cultured under hypoxic conditions (static cultivated cells) in comparison to normally cultured cells (shaken cultivated cells). (B) Specific productivity of CHO-DG44-SEAP and CHO-DG44-5HRE-SEAP cells cultured in ultra-high cell density compared to normally cultured cells (normal cell density). (C+D) Viable cell density (C) and viability (D) of CHO-DG44-5HRE-SEAP cells during fed-batch fermentation. Temp shift = temperature shift (no oxygen shift) (grey line); Temp+O₂ shift = temperature and oxygen shift (black line). (E) SEAP concentration during fed-batch fermentation secreted by CHO-DG44-5HRE-SEAP cells with and without hypoxic shift to 1 % O₂ (oxygen shift). Temp shift = temperature shift (no oxygen shift) (grey line); Temp+O₂ shift = temperature and oxygen shift (black line). (F) Specific productivity of CHO-DG44-5HRE-SEAP cells in average before the temperature and oxygen shift and afterwards. Temp shift = temperature shift (no oxygen shift) (grey bars); Temp+O₂ shift = temperature and oxygen shift (black bars). Statistical analysis was conducted using one-way ANOVA with Bonferroni correction [n = 3 replicates; Mean ± SD; * = p < 0.05; ** = p < 0.01; n.s. = not significant].

### Detailed description of the invention

In a first aspect, the invention relates to a method for producing a biomolecule by a cell cultured *in vitro,* the method comprising the steps of:
(a) propagating the cell in a cell culture, wherein the cell is transformed with a vector, wherein the vector comprises
   (i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
   (ii) a promoter that is active in the cell, wherein the promoter is operably linked to the HRE;
   (iii) a nucleic acid sequence encoding the biomolecule, wherein the nucleic acid sequence is operably linked to the promoter;
   and
(b) isolating the biomolecule from the cell culture.

The term "biomolecule" as used herein refers to any compound which is inherently biological in nature and suitable to be produced by a cell and harvested from the cell or from the cell's culture medium. Preferably, the biomolecule is a biopharmaceutical, i.e. a pharmaceutical which is inherently biological in nature and manufactured by means of a biotechnological process. The biopharmaceutical may be a therapeutic, prophylactic or diagnostic compound. The biopharmaceutical may be, for example, an antibody, a cytokine, a hormone, an enzyme or a vaccine. The biopharmaceutical may also be a virus or a coding or non-coding RNA molecule.

The cell is a cell that is suitable for producing the biomolecule of interest when the cell is cultured *in vitro.* The cell can also be referred to as "producer cell". For biomolecule production, the cell is provided with the nucleic acid sequence encoding the biomolecule and propagated in a cell culture, i.e. grown and proliferated under controlled conditions. The cell culture may be a suspension cell culture or an adherent cell culture. Preferably, the cell culture is a suspension cell culture. Besides cells that naturally grow in suspension, there are also cell lines that have been modified to be able to survive in suspension cell cultures so they can be grown to higher cell densities than their adherent culture would allow.

According to the invention, the nucleic acid sequence encoding the biomolecule is part of the vector with which the cell is transformed.

The term "vector" as used herein refers to a recombinant DNA molecule that is used to introduce genetic material into the cell. The vector may be, for example, an expression vector or a viral vector. The cell is transformed with the vector so that the vector is present in the cell. Methods for transforming the cell with the vector are known in the art and include, for example, transfection of the cell. Transfection is an easy and fast method to transform the cell with the vector.

The vector provides a hypoxia-inducible expression system. By transforming the cell with the vector, the cell is rendered hypoxia-inducible, i.e. it can be referred to as a hypoxia-inducible cell.

The vector comprises at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences.

Hypoxia-responsive elements (HREs), also referred to as hypoxia response elements, are regulatory enhancer elements that have been identified in the promoter region of numerous oxygen-responsive genes such as the VEGF-A, EPO, PGK1, Ldha, ALDA and GAPDH genes. The HREs of the different genes differ in their nucleotide sequences. The HREs serve as the binding site for hypoxia-inducible factors (HIFs), which are transcription factors that mediate the adaptation of cells to low oxygen levels (hypoxia) by enhancing the transcription of the respective genes. Transcription factors generally enhance the transcription of a gene by binding to a specific DNA sequence such as a responsive element.

HIFs are heterodimeric proteins comprising a hypoxia-inducible α subunit with three isoforms (HIF-1a, HIF-2a and HIF-3a) and a constitutively expressed β subunit with two isoforms (HIP-1β and HIP-2β). HIP-1β is also known as aryl hydrocarbon receptor nuclear translocator (ARNT). In short, under normal oxygen conditions (normoxia), i.e. under the presence of sufficient oxygen, the α subunit becomes hydroxylated and ubiquitylated, so that it is degraded by the proteasome. In case of hypoxia, i.e. in case of oxygen deficiency, the α subunit is not hydroxylated and ubiquitylated and is thus stabilized and binds to the β subunit to form a heterodimer. The heterodimer binds to HREs in the promoter/enhancer region of target genes, enhancing the transcription of the genes.

The Von Hippel-Lindau protein (VHL protein), also known as Von Hippel-Lindau tumor suppressor, ubiquitylates the α subunit of HIFs and thus marks the α subunit for degradation. A reduction or loss of VHL protein activity results in an increased amount of HIF α subunit and thus in an increased transcription level of HRE-regulated genes.

The inventors found that HIF-1α, HIP-1β and the VHL protein, which are the key players of the hypoxia sensing pathway, are also present in Chinese hamster ovary (CHO) cells which are the most commonly used mammalian cells for the production of biomolecules. The inventors also found that crucial protein domains of HIF-1α such as the DNA binding domain are conserved in the human and hamster HIF-1α proteins.

The inventors then found that the HRE sequence of the vascular endothelial growth factor A (VEGF-A) gene can be exploited for increasing the production of a biomolecule under hypoxic conditions. To do so, at least one HRE of the VEGF-A gene is operably combined with a suitable promoter and the nucleic acid sequence encoding the biomolecule. This leads to an increased transcription of the nucleic acid sequence encoding the biomolecule under conditions of oxygen deficiency (i.e. under hypoxic conditions) and thus to an increased production of the biomolecule under hypoxic conditions. The increase of biomolecule production under hypoxic conditions is particularly advantageous for cell cultures with high cell densities, in which low levels of oxygen (hypoxic conditions) are inherently present. HREs of the VEGF-A gene have been identified in various organisms such as in human, mouse, rat, hamster, dog and cattle.

The sequence of SEQ ID NO.: 1 is the HRE sequence of the human VEGF-A gene:
CCACAGTGCATACGTGGGCTCCAACAGGTCCTCTT (SEQ ID NO.: 1).

The VEGF-A gene of *Canis lupus familiaris* (dog) and the VEGF-A gene of Bos *taurus* (cattle) are regulated by the same HRE sequence as the human VEGF-A gene.

The sequence of SEQ ID NO.: 2 is the HRE sequence of the *Mus musculus* (house mouse) VEGF-A gene:
ACACAGTGCATACGTGGGTTTCCACAGGTCGTCTC (SEQ ID NO.: 2).

The sequence of SEQ ID NO.: 3 is the HRE sequence of the *Rattus norvegicus* (Norway rat) VEGF-A gene:
CCACAGTGCATACGTGGGCTTCCACAGGTCGTCTC (SEQ ID NO.: 3).

The sequence of SEQ ID NO.: 4 is the HRE sequence of the *Cricetulus griseus* (Chinese hamster) VEGF-A gene:
CCACAGTGCATACGTGGGCTTCCACAGGTCCTCTT (SEQ ID NO.: 4).

All sequences are indicated in 5' -> 3' direction.

A functional variant of any one of the sequences of SEQ ID NO.s: 1-4 is a variant that has one or more alterations in the sequence, wherein the alterations do not alter the ability of the sequence to induce gene expression in response to hypoxia.

The alterations are preferably nucleotide exchanges, but may also be nucleotide insertions or nucleotide deletions.

By comparing the HRE of the erythropoietin (EPO) and the VEGF-A gene in CHO cells, the inventors found that the HRE of the VEGF-A gene is superior and presents the most potent enhancer for biomolecule production in CHO cells when the cells are induced by hypoxic conditions. The inventors also found that the use of the HRE sequence of the human phosphoglycerate kinase 1 (PGK1) gene does not result in a hypoxia-inducible expression system in CHO cells.

The vector further comprises a promoter that is active in the cell, wherein the promoter is operably linked to the HRE. The promoter drives the transcription of the nucleic acid sequence encoding the biomolecule. The promoter can be any promoter that is active in the cell and is thus primarily selected according to the type of the cell that is used for the production of the biomolecule. When selecting the promoter, further known considerations can be taken into account, such as the type of biomolecule that is to be produced and the level of induction of transcription of the nucleic acid sequence encoding the biomolecule. The promoter can be an endogenous promoter or an exogenous promoter. The promoter can be a natural promoter, such as a natural viral promoter, or an engineered (synthetic) promoter.

Two nucleic acid sequences are "operably linked" when one of the nucleic acid sequences is placed into a functional relationship with the other nucleic acid sequence. The term "operably linked" is thus synonymous to the term "functionally linked". In most cases, two nucleic acid sequences that are operably linked are contiguous. However, enhancers, for example, do not have to be contiguous to another nucleic acid sequence in order to be operably linked thereto.

The promoter is operably linked to the HRE, i.e. the promoter is placed into a functional relationship with the HRE. In other words, the promoter and the HRE are arranged in the vector in a manner that allows a functional relationship of the promoter with the HRE, so that the promoter is induced when a HIF heterodimer binds to the HRE.

The vector further comprises a nucleic acid sequence encoding the biomolecule, wherein the nucleic acid sequence is operably linked to the promoter. In other words, the nucleic acid sequence encoding the biomolecule and the promoter are arranged in the vector in a manner that allows a functional relationship of the nucleic acid sequence with the promoter, so that the promoter can drive the transcription of the nucleic acid sequence. For example, as is known in the art, the promoter needs to be arranged in an appropriate orientation relative to the nucleic acid sequence. The transcription of the nucleic acid sequence encoding the biomolecule leads to the production of the biomolecule by the cell.

The nucleic acid sequence encoding the biomolecule can also be referred to as the gene of interest (Gol).

After the step of propagating the cell in the cell culture, which is the step in which the biomolecule is produced, the biomolecule is isolated from the cell culture. Suitable ways of isolating the biomolecule from the cell culture are known and will be selected according to the type of biomolecule produced. For example, if the biomolecule is a recombinant protein that is secreted into the cell culture medium, i.e. into the medium in which the cell is propagated, the protein can be isolated from the cell culture by separating the medium from the cells (e.g. by centrifugation) and by subsequently purifying the protein from the medium (e.g. by chromatography).

Biomolecule production in cell cultures with high cell densities is limited by oxygen deficiency. Oxygen deficiency naturally occurs in the cell culture when the cell density increases to a level at which oxygen supply becomes insufficient. In the prior art, this limitation could only be improved to a very limited extent due to poor oxygen distribution as well as limited oxygen saturation in the cell cultures. The method of the invention solves this problem by transforming the cells with a vector that leads to an increased production of the biomolecule (i.e. the product) under conditions of oxygen deficiency. The cells show improved productivity, despite higher cell densities and lower levels of available oxygen, which allows using more intensive cell culture processes such as perfusion cell cultures for biomolecule production. The method of the invention thus exploits hypoxia to significantly increase biomolecule production.

The inventors confirmed that the hypoxia-inducible cells that were generated for the method of the invention did not express the unstable HIF-1α protein under normoxic conditions (40-60 % O₂). Low levels of the HIF-1α protein were found during cultivation of the cells at 5 % O₂ and higher levels (i.e. a stabilization) of the HIF-1a protein were found at 1 % O₂. In accordance with these findings, the amount of biomolecule produced was increased during cultivation of the cells at 5 % O₂ compared to their cultivation at 40 % O₂ or 60 % O₂. The amount of biomolecule produced was further massively increased during cultivation of the cells at 1 % O₂ compared to their cultivation at 5 % O₂. The amount of biomolecule produced by the hypoxia-inducible cells was nearly 10-fold higher than the amount produced by non-hypoxia-inducible control cells under hypoxic conditions. Taken together, these data confirm the successful establishment of a hypoxia-inducible vector system to increase biomolecule production under conditions of oxygen deficiency.

The method of the invention results in increased cellular productivity and increased product yield while product quality is maintained or improved. This increases the efficiency of the production process and reduces the costs for biomolecule production. The method of the invention also renders the production process more robust, because the previously negative consequences of decreasing oxygen levels are overcome, even turned around, leading to an improved reproducibility and an increased predictability of the biomolecule production process.

The method of the invention allows cells to be cultured at high cell densities while improving cellular productivity and maintaining and/or improving product quality under conditions of oxygen deficiency. Cell cultures with high cell densities are, for example, perfusion cell cultures, batch cell cultures, fed-batch cell cultures or N-1 fed-batch cell cultures. Cell cultures with high cell densities save production time and lower the costs for biomolecule production since the volume of the cell culture can be reduced. A reduced cell culture volume leads to a reduction of cell culture medium and feeds needed, to space saving, waste reduction and a lower energy demand for tempering the cell culture at a suitable temperature such as 37 °C.

In addition, the method of the invention allows the reduction of the gassing rate of the cell culture (with compressed air or pure oxygen) or even the elimination of gassing with pure oxygen, which saves oxygen and thereby further reduces the costs for biomolecule production. Pure oxygen gassing also has a certain toxicity for commonly used CHO cells. The method of the invention also allows the reduction of the stirring speed, which significantly reduces shear stress acting on the cells. The reduction of the shear stress leads to an improvement in cellular parameters such as cell growth, cell viability and cellular productivity. The improvement in these parameters can simplify the isolation and purification of the biomolecule produced.

Further, the method of the invention can lead to a reduction of a possible aggregation of the biomolecule at interphases, thereby improving product quality and leading to a reduction in the costs and workload of downstream processing.

The method of the invention also allows to establish a novel biphasic production process. Conventionally, during a fed-batch process, a temperature shift is applied to the cell culture to ensure maximum productivity. Standard industrial fed-batch processes apply a shift of temperature from 37 °C to a lower temperature such as 34 °C after the cells have reached the static cultivation phase. In this way, the longevity of the production process can be enhanced and the final product titer can be increased as the temperature reduction leads to a favorable redistribution of nutrients for biomolecule production. In addition to the temperature shift or instead of the temperature shift, the method of the invention allows applying an oxygen shift from a normoxic level (e.g. 40 % or 60 % oxygen) to a hypoxic (e.g. 5 % or 1 % oxygen), by which an induction of biomolecule production can be achieved at any cell density, i.e. at any time point of the production process. This requires that the oxygen level in the cell culture can be tightly controlled, which is usually the case when biomolecules are produced. The oxygen shift preferably is applied after the cells have reached the static cultivation phase. The oxygen shift can be applied at the same time as the temperature shift.

The inventors found that there is no significant influence of oxygen levels as low as 1 % O₂ on cell viability when the oxygen shift to 1 % is performed in parallel with (i.e. at the same time as) the temperature shift to 34 °C after the cells have reached the static cultivation phase.

The inventors further found that after shifting the temperature or after shifting both the temperature and the oxygen level, biomolecule production was induced in both hypoxia-inducible and conventional non-hypoxia-inducible cells, however, to a significant stronger extent in hypoxia-inducible cells which benefit from the reduction of the oxygen level. A nearly 2-fold increase of specific productivity compared to conventional non-hypoxia-inducible cells could be achieved by exploiting hypoxia in this manner.

In a preferred embodiment, the promoter is upstream of the nucleic acid sequence encoding the biomolecule. The distance between the promoter and the nucleic acid sequence encoding the biomolecule can be, for example, 0 to about 100 base pairs. In case the distance is 0 base pairs, the promoter is directly upstream of the nucleic acid sequence encoding the biomolecule.

In a further preferred embodiment, the promoter is directly upstream of the nucleic acid sequence encoding the biomolecule. In other words, the 3' end of the promoter sequence is directly adjacent to the 5' end of the nucleic acid sequence encoding the biomolecule. Alternatively, the promoter may be located further upstream of the nucleic acid sequence encoding the biomolecule.

In a preferred embodiment, the HRE is upstream of the promoter. The distance between the HRE and the promoter can be, for example, 0 to about 100 base pairs. In case the distance is 0 base pairs, the HRE is directly upstream of the promoter.

In a further preferred embodiment, the HRE is directly upstream of the promoter. In other words, the 3' end of the HRE sequence is directly adjacent to the 5' end of the promoter sequence. Alternatively, the HRE may be located further upstream of the promoter or downstream of the nucleic acid sequence encoding the biomolecule.

In a particularly preferred embodiment, the promoter is directly upstream of the nucleic acid sequence encoding the biomolecule and the HRE is directly upstream of the promoter.

In a preferred embodiment, the HRE is present in more than one copy. Accordingly, in a preferred embodiment, the vector comprises at least two HREs arranged adjacent to one another, wherein the number of HREs preferably is between 2 and 20, further preferred between 2 and 10, further preferred between 2 and 8, most preferred 5. When using more than one copy of the HRE sequence, the inventors observed a positive correlation between HRE copy number and response in terms of biomolecule production. Thus, it is preferred to use a vector that comprises at least two HREs. More than 20 copies of the HRE sequence would lead to an unnecessarily large vector. Therefore, it is preferred that the vector comprises between 2 and 20 HREs. In CHO cells, the correlation between HRE copy number and biomolecule production was found to be saturated between 5 and 8 HRE repetitions. Overall, the inventors found that 5 copies of the HRE gave the best results in terms of induction of biomolecule production under conditions of oxygen deficiency.

The term "arranged adjacent to one another" refers to a distance of 0 to about 100 base pairs between two adjacent HREs. The distance (spacing) between two adjacent HREs preferably is between 0 and 50 base pairs, further preferred between 0 and 30 base pairs, further preferred between 0 and 20 base pairs, further preferred between 0 and 10 base pairs, most preferred 6 base pairs. In case the distance is 0 base pairs, the two HREs are arranged directly adjacent to one another. The HRE copies do not need to be directly adjacent to one another. For example, the inventors used vectors with different HRE copy numbers with a distance of six base pairs between adjacent HREs. A distance of more than 50 base pairs would lead to an unnecessarily large vector. Therefore, it is preferred that the distance between two adjacent HREs is between 0 and 50 base pairs.

The cell may be any cell that is suitable for the biotechnological production of the biomolecule of interest. The cell preferably is a eukaryotic cell.

The cell may be a human cell or a non-human cell. Many human and non-human cell lines that are suitable for the biotechnological production of biomolecules are known. Among the non-human cell-lines, CHO cells and duck embryo cells are of particular importance. CHO cells are the most commonly used mammalian cells for the industrial production of recombinant therapeutic proteins such as antibodies. Duck embryo cells are particularly useful for the production of virus-based vaccines.

In a preferred embodiment, the cell is a Chinese hamster ovary (CHO) cell, a derivative of a CHO cell, a SL-29 cell, a duck embryo cell, a QT6 cell, a Vero cell, a NIH/3T3 cell, a Baby Hamster Kidney 21 (BHK-21) cell, a Sp2/0-Ag14 cell, a BTI-Tn-5B1-4 cell, a hybridoma cell, a HeLa cell, a Human Embryonic Kidney 293 (HEK293) cell, a derivative of a HEK293 cell, a CAP cell or a CAP-T cell.

In a preferred embodiment, the derivative of the CHO cell is a CHO-K1 cell, a CHO-EBNA cell, a CHO-EBNA GS cell, a CHO-DG44 cell, a CHO-DXB11 cell or a CHO-S cell. CHO-K1 and CHO-DG44 cells are the prevailing derivatives used in industry. CHO-EBNA cells are CHO-K1 cells that are engineered to express the gene encoding Epstein-Barr virus nuclear antigen-1 (EBNA-1). CHO-EBNA GS cells are CHO-K1 cells that are engineered to express the gene encoding EBNA-1 and to express the gene encoding glutamine synthetase (GS).

In a preferred embodiment, the derivative of the HEK293 cell is a HEK293F cell or a HEK293S cell. In contrast to HEK293 cells, HEK293F and HEK293S cells are adapted to grow in suspension. HEK293F cells are known for their fast growth and high transfection efficiency. Further derivatives of the HEK293 cell are, for example, a HEK293T cell (ATCC number: CRL-3216), a HEK293FT cell (a fast growing variant of HEK293T), a HEK293E cell and a HEK293SG cell.

A hybridoma cell is an immortalized cell derived from the fusion of a B lymphoblast with a myeloma fusion partner. The generation of a hybridoma cell starts by immunizing a mouse with a target antigen, thereby eliciting an immune response. The B lymphocytes that are taken from the immunized mouse spleen produce antibodies to the antigen. Each B lymphocyte is then fused with an immortal myeloma cell line to produce a hybrid cell line called a hybridoma, The hybridoma cell has both the antibody-producing ability of the B lymphocyte and the longevity and reproductivity of the myeloma, allowing the production of large quantities of monoclonal antibodies. Sp2/0-Ag14 is an example of a hybridoma fusion partner myeloma cell line.

An overview of the preferred cell lines is given in Table 1.

**Table 1: Preferred cell lines for the production of biomolecules**

| Cell line | Alternative name(s) of the cell line | Species | American Type Culture Collection (ATCC) number, where applicable |
|---|---|---|---|
| CHO | Chinese hamster ovary | *Cricetulus bara-bensis griseus* | |
| CHO-K1 | Chinese hamster ovary K1; | *Cricetulus bara-bensis griseus* | CCL-61 |
| | CHO K1; | | |
| | CHOK1; | | |
| | CHO cell clone K1; | | |
| | GM15452 | | |
| CHO-EBNA | Chinese hamster ovary EBNA; CHO EBNA | *Cricetulus bara-bensis griseus* | |
| CHO-EBNA GS | Chinese hamster ovary EBNA GS; CHO EBNA GS | *Cricetulus bara-bensis griseus* | |
| CHO-DG44 | Chinese hamster ovary DG44; | *Cricetulus bara-bensis griseus* | |
| | CHO DG44; | | |
| | CHO-DG44 (DHFR-); | | |
| | CHO DG-44; | | |
| | DG-44; | | |
| | DG44 | | |
| CHO-DXB11 | Chinese hamster ovary DXB11; | *Cricetulus bara-bensis griseus* | CRL-9096 |
| | CHO-DUKX; | | |
| | CHO DXB11; | | |
| | CHO-DUX-B11; | | |
| | CHO (Dux B11); | | |
| | CHO-DUK-XB11; | | |
| | DUK-XB11; | | |
| | CHO-DUKX B11; | | |
| | CHO-DUKXB11; | | |
| | DUKX-B11; | | |
| | DUX B11; | | |
| | DUXB11; | | |
| | DXB-11; | | |
| | DXB11; | | |
| | DUKX-CHO; | | |
| | DUKX; | | |
| | CHO-DHFR -; | | |
| | CHO/dhFr-; | | |
| | CHO duk-; | | |
| | CHO B11; | | |
| | B11 | | |
| CHO-S | Chinese hamster ovary S; CHOS | *Cricetulus bara-bensis griseus* | |
| SL-29 | | *Gallus gallus* | CRL-1590 |
| Duck embryo | | *Anas platyrhyn-chus domesticus* | CCL-141 |
| QT6 | Quail Tumor 6; QT-6; | *Coturnix coturnix japonica* | CRL-1708 |
| | QT 6; | | |
| | CHQC-QT6 | | |
| Vero | VERO; | *Cercopithecus sabaeus* | CCL-81 |
| | VeroCCL81; | | |
| | Vero 81; | | |
| | Verda reno | | |
| NIH/3T3 | | *Mus musculus* | CRL-1658 |
| BHK-21 | Baby Hamster Kidney 21; | *Mesocricetus au-ratus* | CCL-10 |
| | BHK 21; | | |
| | BHK21; | | |
| | Baby Hamster Kidney-21 | | |
| Sp2/0-Ag14 | SP2/0-Ag14; | *Mus musculus* | CRL-1581 |
| | SP2/0-AG14; | | |
| | SP2/0-ag14; | | |
| | Sp2/O-Ag14; | | |
| | SP2/O-Ag14; | | |
| | Sp2/0-Ag-14; | | |
| | SP2-0-Ag14; | | |
| | SP2/0 Ag-14; | | |
| | SP-2/0-AG14; | | |
| | Sp 2/0-Ag 14 | | |
| BTI-Tn-5B1-4 | High Five; | *Trichoplusia ni* | |
| | High 5; | | |
| | High-5; | | |
| | High5; | | |
| | Hi-five; | | |
| | Hi-5; | | |
| | Hi5; | | |
| | BTI-TN-5B1-4; | | |
| | BTI-TN5B1-4; | | |
| | BTI-Tn5B14; | | |
| | BTI-Tn 5B1-4; | | |
| | Tn-5B1-4; | | |
| | Tn 5B1-4; | | |
| | Tn5 B1-4; | | |
| | Tn5B1-4; | | |
| | TN5B14; | | |
| | TnH5; | | |
| | Tn-5 | | |
| HeLa | HELA; | *Homo sapiens* | CCL-2 |
| | Hela; | | |
| | He La; | | |
| | He-La; | | |
| | Henrietta Lacks; | | |
| | Helacyton gartleri | | |
| HEK293 | Human Embryonic | *Homo sapiens* | CRL-1573 |
| | Kidney 293; | | |
| | Hek293; | | |
| | HEK-293; | | |
| | HEK/293; | | |
| | HEK 293; | | |
| | 293; | | |
| | 293 HEK | | |
| HEK293F | Human Embryonic | *Homo sapiens* | |
| | Kidney 293F; | | |
| | HEK-293-F; | | |
| | HEK 293-F; | | |
| | HEK-293F; | | |
| | HEK293F; | | |
| | 293-F; | | |
| | 293 F; | | |
| | 293F | | |
| HEK293S | Human Embryonic Kidney 293S; 293S | *Homo sapiens* | |
| CAP | CEVEC's Amnio-cyte Production; N52.E6 | *Homo sapiens* | |
| CAP-T | CEVEC's Amnio-cyte Production T | *Homo sapiens* | |

The cell preferably is a mammalian cell. In another embodiment, the cell preferably is an insect cell.

The promoter preferably is a complete promoter. The method of the invention is suitable for using a complete promoter. Complete promoters are generally used when producing a biomolecule by a cell cultured *in vitro.*

In a preferred embodiment, the promoter is selected from the group consisting of cytomegalovirus (CMV) promoter, simian virus 40 early (SV40E) promoter, elongation factor 1 alpha (EF-1α) promoter, hEF1-HTLV promoter, T7 RNA polymerase and SP6 RNA polymerase (T7/SP6) promoter, ubiquitin C (UBC) promoter, U6 promoter, ferritin heavy chain (FerH) promoter, ferritin light chain (FerL) promoter, phosphoglycerate kinase (PGK) promoter, thyroxine-binding globulin (TBG) promoter, albumin (Alb) promoter and alpha-fetoprotein (AFP) promoter.

The EF-1α promoter, which is also referred to as EF1 promoter, preferably is the human EF-1α (hEF1) promoter.

The hEF1-HTLV promoter is a composite promoter composed of the human elongation factor 1 alpha (EF-1α) core promoter and the R segment and part of the U5 sequence (R-U5') of the Human T-Cell Leukemia Virus (HTLV) Type 1 Long Terminal Repeat.

The T7/SP6 promoter is a combination of two promoters, namely the T7 RNA polymerase promoter and the SP6 RNA polymerase promoter.

The U6 promoter is an RNA polymerase III promoter that allows expression of small non-coding RNA molecules such as shRNAs or miRNAs with precise 5' and 3' sequences (i.e. no 5' cap or 3' polyA tail).

The promoter may also be a minimal promoter, i.e. a promoter that consists of the minimal sequence that can act as an effective promoter. The minimal promoter can be, for example, a minimal CMV promoter.

The vector may further comprise one or more regulatory elements other than HRE, in particular one or more enhancers, that are operably linked to the promoter and regulate the transcription of the nucleic acid sequence encoding the biomolecule. Examples of suitable enhancers include hepatitis B virus enhancers (HBVE), tyrosinase (Tyr) enhancer and aldehyde dehydrogenase (AldA) enhancer.

The vector may further comprise a nucleic acid sequence encoding a selection marker that allows selecting the cells that have been stably transformed with the vector. Suitable selection markers depend on the cell used and are known in the art. For example, if the cell is a dihydrofolate reductase (DHFR) deficient cell (e.g. a CHO-DG44 cell), DHFR is a suitable selection marker. In this case, the vector preferably further comprises a nucleic acid sequence encoding DHFR.

In a preferred embodiment, the cell is further transformed with a nucleic acid sequence comprising a region encoding an RNA interference (RNAi) molecule that prevents or reduces expression of Von Hippel-Lindau protein, wherein the RNAi molecule is a small interfering RNA (siRNA), a short hairpin RNA (shRNA) or a microRNA (miRNA), and wherein the region encoding the RNAi molecule is operably linked to a promoter that is active in the cell.

As mentioned above, the Von Hippel-Lindau protein (VHL protein) ubiquitylates the α subunit of HIFs and thus marks the α subunit for degradation. Preventing or reducing the expression of the VHL protein results in an increased amount of HIF α subunit and thus in an increased expression level of the HRE-regulated nucleic acid sequence encoding the biomolecule. For this reason, further transforming the cell with the nucleic acid sequence that comprises the region encoding the RNAi molecule that prevents or reduces expression of the VHL protein is of advantage for further increasing the production of the biomolecule.

The following sequences are examples of sequences of siRNA that prevents or reduces the expression of the VHL protein:
UGGUCAAGCCUGAGAACUAUU (SEQ ID NO.: 56)
CCAAAUGUGCGGAAGGACAUU (SEQ ID NO.: 57)
CCGGAAGGCAGCUGGUCAAUU (SEQ ID NO.: 58)

All sequences are indicated in 5' -> 3' direction.

RNA interference is a naturally occurring process in which RNA molecules are involved in sequence-specific suppression of gene expression. RNAi molecules suppress (i.e. prevent or reduce) gene expression by mRNA degradation which prevents the translation of the mRNA into the respective protein. The RNAi molecule is a siRNA, a shRNA or a miRNA, all of which are small non-coding RNA molecules.

siRNA molecules are double-stranded RNA molecules of about 19-23 base pair nucleotides in length. shRNA molecules have a hairpin like stem-loop structure since they consist of two complementary RNA sequences of about 19-22 nucleotides in length that are linked by a short loop of 4-11 nucleotides. Both siRNA and shRNA molecules are highly specific, having only one mRNA target.

miRNA molecules are single stranded RNA molecules of about 20-24 nucleotides in length and have multiple mRNA targets.

To enable transcription of the RNAi molecule, the region encoding the RNAi molecule is operably linked to a promoter that is active in the cell. The promoter drives the transcription of the RNAi molecule. The promoter preferably is comprised by the nucleic acid sequence that comprises the region encoding the RNAi molecule. The promoter is primarily selected according to the type of the cell that is used for the production of the biomolecule and for its suitability regarding the transcription of the RNAi molecule. All promoters mentioned above with respect to the nucleic acid sequence encoding the biomolecule can be used as promoters for the region encoding the RNAi molecule. The promoter preferably is the U6 promoter. In case the RNAi molecule is a miRNA, the CMV promoter is also preferred.

The nucleic acid sequence that comprises the region encoding the RNAi molecule preferably is part of a vector. The vector can be the same vector as the vector comprising the HRE, the promoter and the nucleic acid sequence encoding the biomolecule. Accordingly, in a preferred embodiment, the nucleic acid sequence comprising the region encoding the RNAi molecule is comprised by the vector. In this case, the vector comprises:
(i) at least one HRE consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
(ii) a first promoter that is active in the cell, wherein the first promoter is operably linked to the HRE;
(iii) a nucleic acid sequence encoding the biomolecule, wherein the nucleic acid sequence is operably linked to the first promoter;
(iv) a nucleic acid sequence comprising
   (a) a second promoter that is active in the cell; and
   (b) a region encoding an RNAi molecule that prevents or reduces expression of Von Hippel-Lindau protein, wherein the RNAi molecule is a siRNA, a shRNA or a miRNA, and wherein the region encoding the RNAi molecule is operably linked to the second promoter.

In an alternative embodiment, the nucleic acid sequence that comprises the region encoding the RNAi molecule can be part of a different vector. This would avoid the need to generate an unnecessarily large vector.

In yet another alternative embodiment, the nucleic acid sequence that comprises the region encoding the RNAi molecule is inserted into the cell's genome. In this way, a modified cell would be provided. This would also avoid the need to generate an unnecessarily large vector.

In a preferred embodiment, the promoter to which the region encoding the RNAi molecule is operably linked, is operably linked to at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences. In this embodiment, the production of the RNAi molecule is regulated in the same manner as the production of the biomolecule, i.e. its production is increased under hypoxic conditions. Accordingly, hypoxic conditions lead to (i) an increase of the transcription of the nucleic acid sequence encoding the biomolecule, and (ii) an increased amount of HIF α subunit by increasing the transcription of the region encoding the RNAi molecule that prevents or reduces the expression of the Von Hippel-Lindau protein.

The increased amount of HIF α subunit leads to an increased formation of heterodimers of the HIF α subunit and the HIF β subunit. The heterodimers bind to the HRE(s), thereby further enhancing the transcription of the nucleic acid sequence encoding the biomolecule as well as the transcription of the region encoding the RNAi molecule. Accordingly, in this way, a positive feedback loop is established that has an enhancing effect on biomolecule production under hypoxic conditions.

The positive feedback loop leads to a higher production yield at a given level of oxygen. This also means that the same production yield can be reached at higher levels of oxygen in the cell culture, for example at 10 % oxygen rather than at 1 % oxygen. This is of advantage for cells that show a reduced viability at extremely low oxygen levels such as 1 % oxygen.

The at least one HRE preferably is comprised by the nucleic acid sequence that comprises the region encoding the RNAi molecule. The nucleic acid sequence comprising the region encoding the RNAi molecule thus preferably comprises (a) at least one HRE consisting of a nucleic acid sequence selected from the group consisting of SEQ ID NO.: 1, SEQ ID NO.: 2, SEQ ID NO.: 3, SEQ ID NO.: 4 and functional variants of any one of these sequences; (b) a promoter that is active in the cell, wherein the promoter is operably linked to the HRE; (c) the region encoding the RNAi molecule, wherein the region encoding the RNAi molecule is operably linked to the promoter.

The embodiments described above with respect to the HRE that controls the transcription of the nucleic acid sequence encoding the biomolecule likewise apply to the HRE that controls the transcription of the region encoding the RNAi molecule.

In a preferred embodiment, the promoter to which the region encoding the RNAi molecule is operably linked is directly upstream of the region encoding the RNAi molecule and the HRE is directly upstream of the promoter.

In another embodiment, the nucleic acid sequence encoding the biomolecule additionally encodes the RNAi molecule (i.e. the nucleic acid sequence encoding the biomolecule additionally comprises the region that encodes the RNAi molecule) and is transcribed into an mRNA transcript that harbours a respective splicing site so that both the biomolecule and the RNAi molecule will be generated from the mRNA transcript by splicing. The splicing site may be, for example, a pre-tRNA splicing site. In this embodiment, one promoter is sufficient to drive the transcription of both the nucleic acid sequence encoding the biomolecule and the region encoding the RNAi molecule. Likewise, one HRE or one stretch of multiple HREs is sufficient to control the transcription of both the nucleic acid sequence encoding the biomolecule and the region encoding the RNAi molecule. In this case, the vector comprises:
(i) at least one HRE consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
(ii) a promoter that is active in the cell, wherein the promoter is operably linked to the HRE;
(iii) a nucleic acid sequence encoding the biomolecule and encoding an RNAi molecule that prevents or reduces expression of Von Hippel-Lindau protein, wherein the RNAi molecule is a siRNA, a shRNA or a miRNA, wherein the nucleic acid sequence is operably linked to the promoter.

In a preferred embodiment, the cell is further transformed with a nucleic acid sequence encoding a HIF α subunit, wherein the nucleic acid sequence encoding the HIF α subunit is operably linked to a promoter that is active in the cell. The HIF α subunit preferably is HIF-1α. In this embodiment, the HIF α subunit is overex-pressed in the cell. The increased amount of HIF α subunit leads to an increased formation of heterodimers of the HIF α subunit and the HIF β subunit. The heterodimers bind to the HRE(s), thereby further enhancing the transcription of the nucleic acid sequence encoding the biomolecule. The qualitative effect of overexpressing the HIF α subunit is thus comparable to the effect of the RNAi molecule that prevents or reduces the expression of the Von Hippel-Lindau protein in the cell. Overexpressing the HIF α subunit may thus be applied as an alternative to expressing the RNAi molecule that prevents or reduces the expression of the Von Hippel-Lindau protein.

The embodiments described above with respect to the region encoding the RNAi molecule that prevents or reduces the expression of the Von Hippel-Lindau protein or with respect to the nucleic acid sequence that comprises the region encoding the RNAi molecule likewise apply to the nucleic acid sequence encoding the HIF α subunit.

In particular, in a preferred embodiment, the promoter to which the nucleic acid sequence encoding the HIF α subunit is operably linked, is operably linked to at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences. In this embodiment, the expression of the HIF α subunit is regulated in the same manner as the production of the biomolecule, i.e. its expression is increased under hypoxic conditions. Accordingly, hypoxic conditions lead to (i) an increase of the transcription of the nucleic acid sequence encoding the biomolecule, and (ii) an increased amount of HIF α subunit. The increased amount of HIF α subunit leads to an increased formation of heterodimers of the HIF α subunit and the HIF β subunit. The heterodimers bind to the HRE(s), thereby further enhancing the transcription of the nucleic acid sequence encoding the biomolecule as well as the transcription of the nucleic acid sequence encoding the HIF α subunit. Accordingly, in this way, a positive feedback loop is established that has an enhancing effect on biomolecule production under hypoxic conditions.

The positive feedback loop leads to a higher production yield at a given level of oxygen. This also means that the same production yield can be reached at higher levels of oxygen in the cell culture.

The embodiments described above with respect to the HRE that controls the transcription of the nucleic acid sequence encoding the biomolecule likewise apply to the HRE that controls the transcription of the nucleic acid sequence encoding the HIF α subunit.

In a preferred embodiment, the promoter to which the nucleic acid sequence encoding the HIF α subunit is operably linked is directly upstream of the nucleic acid sequence encoding the HIF α subunit and the HRE is directly upstream of the promoter.

Overexpression of the HIF α subunit may also be used in combination with expression of the RNAi molecule that prevents or reduces the expression of the Von Hippel-Lindau protein. Accordingly, in a preferred embodiment, the cell is further transformed with both
(i) the nucleic acid sequence comprising the region encoding the RNAi molecule that prevents or reduces expression of Von Hippel-Lindau protein, wherein the RNAi molecule is a siRNA, a shRNA or a miRNA, wherein the region encoding the RNAi molecule is operably linked to a promoter that is active in the cell,
   and
(ii) the nucleic acid sequence encoding the HIF α subunit, wherein the nucleic acid sequence encoding the HIF α subunit is operably linked to a promoter that is active in the cell.

In a preferred embodiment, the biomolecule is a recombinant peptide, a recombinant protein, a recombinant virus, a recombinant virus-like particle or a recombinant mRNA, wherein the recombinant protein preferably is an antibody.

The biomolecule preferably is a biopharmaceutical. Biopharmaceuticals include, for example, recombinant peptides, recombinant proteins, recombinant viruses, recombinant virus-like particles and recombinant mRNAs.

The recombinant protein preferably is an antibody, a derivative of an antibody, a fragment of an antibody, a cytokine, a hormone, a growth factor, a transcription factor, a ribosomal protein, an enzyme or a vaccine.

The recombinant virus preferably is an oncolytic virus or a virus used for gene therapy.

Each of the recombinant virus, the recombinant virus-like particle and the recombinant mRNA may preferably be a vaccine. A virus-like particle (VLP) closely resembles a virus, but is non-infectious because it comprises no viral genetic material. It can be produced by expressing viral structural proteins, which can then self-assemble into the VLP.

The biomolecule may be an amino acid such as, for example, cysteine, serine or threonine.

The biomolecule may be a vitamin.

The biomolecule may be a coding or non-coding RNA molecule. The non-coding RNA molecule may be, for example, a small non-coding RNA (such as, for example, a siRNA, a shRNA or a miRNA), a long non-coding RNA (IncRNA) or an RNA aptamer. The coding RNA molecule may be, for example, a recombinant mRNA. The RNA molecule may be an RNA-based therapeutic.

The biomolecule may be released from the cell into the cell culture medium, i.e. into the medium in which the cell is propagated. The biomolecule may be released from the cell and isolated from the cell culture as part of an extracellular vesicle (EV) that is released from the cell. An example of such a biomolecule is a recombinant membrane protein. The biomolecule may be released from the cell as cargo of an EV that is released from the cell. An example of such an EV is an EV with a coding or non-coding RNA molecule as cargo. The EV may be, for example, a recombinant exosome.

The biomolecule may be a single gene product (e.g. a single protein) or the biomolecule may be composed of more than one gene product. In the latter case, the nucleic acid sequence encoding the biomolecule encodes more than one gene product. For example, in case the biomolecule is a virus-like particle, the nucleic acid sequence encoding the biomolecule encodes more than one viral protein. The number of gene products encoded by the nucleic acid sequence encoding the biomolecule is limited only by the size of nucleic acid that can be inserted into the vector.

The biomolecule may also be a molecule that is not intended for a pharmaceutical use. For example, the biomolecule may be a recombinant protein that can be used as an additive or substitute in food and/or animal feed. An example of such a protein is casein.

In a preferred embodiment, the cell culture is a perfusion cell culture, a batch cell culture or a fed-batch cell culture. These types of cell cultures allow high cell densities, i.e. cell densities of 15x10⁶ cells/ml or more. For example, perfusion cell cultures can have cell densities of 15x10⁶ cells/ml to 100x10⁶ cells/ml. A high cell density cell culture is of advantage for the method of the invention since due to the high cell density, it is typically accompanied by hypoxic conditions, which lead to an increase in the production of the biomolecule. In other words, the method of the invention is particularly beneficial for high cell density cell culture since it exploits high cell density-induced hypoxia.

In perfusion cell culture, cells are retained inside the bioreactor or cells are recycled back to the bioreactor. Fresh cell culture medium is continuously added to the bioreactor and cell-free supernatant comprising the product of interest (i.e. the biomolecule), waste products and depleted cell culture medium is continuously removed from the bioreactor at the same rate. Perfusion cell cultures can have considerably higher cell densities than batch cell cultures.

The inventors performed a mock-perfusion cell culture using the method of the invention by replacing the cell culture medium daily. The inventors surprisingly observed a significantly better performance of the cells in comparison to a normal cell culture of the cells. The increase of the specific productivity of the cells during the mock-perfusion conditions was more than 2-fold compared to the normal culture of the cells.

In batch cell culture, all nutrients are provided at the beginning of the cultivation, i.e. no nutrients are added during the subsequent cultivation process.

In fed-batch cell culture, one or more nutrients are supplied to the bioreactor during the cultivation, while the product of interest remains in the bioreactor until the end of the run.

The fed-batch cell culture preferably is an N-1 fed-batch cell culture. In N-1 fed-batch cell culture, a step of intensification of cell growth is performed in an N-1 bioreactor prior to the cell culture in the production bioreactor (N). This facilitates a higher starting cell density in the production bioreactor and shortens the production bioreactor run time.

In a preferred embodiment, the cell culture has a cell density of at least 15x10⁶ cells/ml, preferably at least 50x10⁶ cells/ml. A cell culture with a cell density of 15x10⁶ cells/ml or more, in particular with a cell density of 50x10⁶ cells/ml or more, typically shows oxygen deficiency, which leads to an increase in the production of the biomolecule.

In a preferred embodiment, the cell culture is exposed to a hypoxic condition, wherein the hypoxic condition preferably is a level of oxygen from 0.1 % to 15 %, further preferred from 0.1 % to 10 %, further preferred from 1 % to 5 %, most preferred 1 %. The level of oxygen can also be referred to as the concentration of oxygen. The hypoxic condition leads to an increase in the production of the biomolecule. The method of the invention allows applying an oxygen shift to the cell culture, namely an oxygen shift from a normoxic condition (e.g. 40 % or 60 % oxygen) to the hypoxic condition (e.g. 5 % or 1 % oxygen), by which biomolecule production will be increased at any cell density, i.e. at any time point of the production process. This requires that the oxygen level in the cell culture can be tightly controlled, which is usually the case when biomolecules are produced. The oxygen shift (hypoxic shift) preferably is applied after the cells have reached the static cultivation phase. The oxygen shift can be applied in combination with the temperature shift that is known from conventional production processes.

The HRE and the cell can be derived from the same species.

Alternatively, the HRE can be from a different species than the cell. An example for such a cross-species embodiment is the use of the human HRE sequence of SEQ ID NO.: 1 in a CHO cell.

In a preferred embodiment, the HRE consists of a nucleic acid sequence selected from the group consisting of SEQ ID NO.: 1, SEQ ID NO.: 2, SEQ ID NO.: 3 and SEQ ID NO.: 4.

In a particularly preferred embodiment, the HRE consists of the nucleic acid sequence of SEQ ID NO.: 1.

In a further preferred embodiment, the HRE consists of the nucleic acid sequence of SEQ ID NO.: 1 and the cell is a CHO cell.

The HRE can consists of a nucleic acid sequence that is a functional variant of any one of SEQ ID NO.: 1, SEQ ID NO.: 2, SEQ ID NO.: 3 and SEQ ID NO.: 4. The functional variant has one or more alterations compared to any one of SEQ ID NO.: 1, SEQ ID NO.: 2, SEQ ID NO.: 3 or SEQ ID NO.: 4, wherein the alterations do not alter the ability of the sequence to induce gene expression in response to hypoxia. The alterations preferably are nucleotide exchanges. The one or more alterations preferably are not in the conserved HIF binding site, which is the most important sequence element of the HRE sequence. Counting the nucleotides in in 5' -> 3' direction, the conserved binding site of HIF is formed by nucleotides 11 to 18 of SEQ ID NO.s: 1-4 (i.e. by the nucleotides at positions 11 to 18) (underlined in Figure 1). Accordingly, the one or more alterations preferably concern nucleotides 1 to 10 and/or nucleotides 19 to 35 of SEQ ID NO.s: 1-4.

In addition to the HIF binding site, the nucleotides at positions 2 to 6 and at positions 24 to 28 may be important for the ability of the sequence to induce gene expression in response to hypoxia. Accordingly, in a preferred embodiment, the one or more alterations concern nucleotide 1, nucleotides 7 to 10, nucleotides 19 to 23 and/or nucleotides 29 to 35 of SEQ ID NO.s: 1-4.

In a particularly preferred embodiment, the one or more alterations are nucleotide exchanges of nucleotide 1, nucleotide 19, nucleotide 21, nucleotide 23, nucleotide 31 and/or nucleotide 35 of SEQ ID NO.s: 1-4. These nucleotides partially vary between SEQ ID NO.: 1, SEQ ID NO.: 2, SEQ ID NO.: 3 and SEQ ID NO.: 4 (Figure 1). In other words, these nucleotides are not conserved. It can thus be reasonably assumed that an exchange of one or more of these nucleotides will not affect the ability of the sequence to induce gene expression in response to hypoxia.

Examples of functional variants of SEQ ID NO.s: 1-4 according to the particularly preferred embodiment described above are given in the following with nucleotide exchanges being underlined. All sequences are indicated in 5' -> 3' direction.

Preferred functional variants of SEQ ID NO.: 1:
ACACAGTGCATACGTGGGCTCCAACAGGTCCTCTT (SEQ ID NO.: 5)
CCACAGTGCATACGTGGGTTCCAACAGGTCCTCTT (SEQ ID NO.: 6)
CCACAGTGCATACGTGGGCTTCAACAGGTCCTCTT (SEQ ID NO.: 7)
CCACAGTGCATACGTGGGCTCCCACAGGTCCTCTT (SEQ ID NO.: 8)
CCACAGTGCATACGTGGGCTCCAACAGGTCGTCTT (SEQ ID NO.: 9)
CCACAGTGCATACGTGGGCTCCAACAGGTCCTCTC (SEQ ID NO.: 10)
CCACAGTGCATACGTGGGCTCCAACAGGTCGTCTC (SEQ ID NO.: 11)
CCACAGTGCATACGTGGGCTCCCACAGGTCGTCTC (SEQ ID NO.: 12)
ACACAGTGCATACGTGGGCTTCCACAGGTCGTCTC (SEQ ID NO.: 13)
SEQ ID NO.: 2
SEQ ID NO.: 3
SEQ ID NO.: 4

Preferred functional variants of SEQ ID NO.: 2:
CCACAGTGCATACGTGGGTTTCCACAGGTCGTCTC (SEQ ID NO.: 14)
ACACAGTGCATACGTGGGCTTCCACAGGTCGTCTC (SEQ ID NO.: 13)
ACACAGTGCATACGTGGGTTCCCACAGGTCGTCTC (SEQ ID NO.: 15)
ACACAGTGCATACGTGGGTTTCAACAGGTCGTCTC (SEQ ID NO.: 16)
ACACAGTGCATACGTGGGTTTCCACAGGTCCTCTC (SEQ ID NO.: 17)
ACACAGTGCATACGTGGGTTTCCACAGGTCGTCTT (SEQ ID NO.: 18)
ACACAGTGCATACGTGGGCTCCCACAGGTCGTCTC (SEQ ID NO.: 19)
ACACAGTGCATACGTGGGTTCCAACAGGTCGTCTC (SEQ ID NO.: 20)
ACACAGTGCATACGTGGGCTCCAACAGGTCGTCTC (SEQ ID NO.: 21)
CCACAGTGCATACGTGGGCTCCAACAGGTCGTCTC (SEQ ID NO.: 11)
ACACAGTGCATACGTGGGTTTCCACAGGTCCTCTT (SEQ ID NO.: 22)
SEQ ID NO.: 1
SEQ ID NO.: 3
SEQ ID NO.: 4

Preferred functional variants of SEQ ID NO.: 3:
ACACAGTGCATACGTGGGCTTCCACAGGTCGTCTC (SEQ ID NO.: 13)
CCACAGTGCATACGTGGGTTTCCACAGGTCGTCTC (SEQ ID NO.: 14)
CCACAGTGCATACGTGGGCTCCCACAGGTCGTCTC (SEQ ID NO.: 12)
CCACAGTGCATACGTGGGCTTCAACAGGTCGTCTC (SEQ ID NO.: 23)
CCACAGTGCATACGTGGGCTTCCACAGGTCCTCTC (SEQ ID NO.: 24)
CCACAGTGCATACGTGGGCTTCCACAGGTCGTCTT (SEQ ID NO.: 25)
CCACAGTGCATACGTGGGCTCCAACAGGTCGTCTC (SEQ ID NO.: 11)
ACACAGTGCATACGTGGGCTCCAACAGGTCGTCTC (SEQ ID NO.: 21)
ACACAGTGCATACGTGGGCTTCCACAGGTCCTCTT (SEQ ID NO.: 26)
SEQ ID NO.: 1
SEQ ID NO.: 2
SEQ ID NO.: 4

Preferred functional variants of SEQ ID NO.: 4:
ACACAGTGCATACGTGGGCTTCCACAGGTCCTCTT (SEQ ID NO.: 26)
CCACAGTGCATACGTGGGTTTCCACAGGTCCTCTT (SEQ ID NO.: 27)
CCACAGTGCATACGTGGGCTCCCACAGGTCCTCTT (SEQ ID NO.: 8)
CCACAGTGCATACGTGGGCTTCAACAGGTCCTCTT (SEQ ID NO.: 7)
CCACAGTGCATACGTGGGCTTCCACAGGTCGTCTT (SEQ ID NO.: 25)
CCACAGTGCATACGTGGGCTTCCACAGGTCCTCTC (SEQ ID NO.: 24)
ACACAGTGCATACGTGGGCTCCAACAGGTCCTCTT (SEQ ID NO.: 5)
ACACAGTGCATACGTGGGCTTCCACAGGTCGTCTC (SEQ ID NO.: 13)
SEQ ID NO.: 1
SEQ ID NO.: 2
SEQ ID NO.: 3

The preferred functional variants of any one of SEQ ID NO.s: 1-4 are also preferred functional variants for the remaining three sequences of SEQ ID NO.s: 1-4. In other words, the preferred functional variants of SEQ ID NO.: 1 are the variants of SEQ ID NO.s: 5-27 and SEQ ID NO.: 2, SEQ ID NO.: 3 and SEQ ID NO.: 4; the preferred functional variants of SEQ ID NO.: 2 are the variants of SEQ ID NO.s: 5-27 and SEQ ID NO.: 1, SEQ ID NO.: 3 and SEQ ID NO.: 4; the preferred functional variants of SEQ ID NO.: 3 are the variants of SEQ ID NO.s: 5-27 and SEQ ID NO.: 1, SEQ ID NO.: 2 and SEQ ID NO.: 4; and the preferred functional variants of SEQ ID NO.: 4 are the variants of SEQ ID NO.s: 5-27 and SEQ ID NO.: 1, SEQ ID NO.: 2 and SEQ ID NO.: 3.

In a second aspect, the present invention relates to a cell for producing a biomolecule *in vitro,* wherein the cell is transformed with a vector, wherein the vector comprises
(i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
(ii) a promoter that is active in the cell, wherein the promoter is operably linked to the HRE;
(iii) a nucleic acid sequence encoding the biomolecule, wherein the nucleic acid sequence is operably linked to the promoter.

The embodiments described above with respect to the cell and the vector used in the method of the invention likewise apply to the cell of the invention. In particular, the cell may be any cell that is suitable for the biotechnological production of the biomolecule of interest. The cell preferably is a eukaryotic cell. The cell may be a human cell or a non-human cell. Many human and non-human cell lines that are suitable for the biotechnological production of biomolecules are known.

Under hypoxic conditions, the cell of the invention increases its production of the biomolecule by increasing the transcription of the nucleic acid sequence encoding the biomolecule.

In a preferred embodiment, the cell is not a tumor cell. The term "tumor cell" as used herein refers to a tumor cell that is transformed with a vector in the context of a gene therapy of a cancer.

In a preferred embodiment, the cell is a Chinese hamster ovary (CHO) cell, a derivative of a CHO cell, a SL-29 cell, a duck embryo cell, a QT6 cell, a Vero cell, a NIH/3T3 cell, a BHK-21 cell, a Sp2/0-Ag14 cell, a BTI-Tn-5B1-4 cell, a hybridoma cell, a HeLa cell, a Human Embryonic Kidney 293 (HEK293) cell, a derivative of a HEK293 cell, a CAP cell or a CAP-T cell, wherein the derivative of the CHO cell preferably is a CHO-K1 cell, a CHO-EBNA cell, a CHO-EBNA GS cell, a CHO-DG44 cell, a CHO-DXB11 cell or a CHO-S cell, and wherein the derivative of the HEK293 cell preferably is a HEK293F cell or a HEK293S cell.

Further aspects of the invention will be apparent to the person skilled in the art by the enclosed description of the examples, in particular the scientific results.

### Examples

### Materials and methods

### Cell culture

CHO (Chinese hamster ovary) cells were cultured in animal component free SFM4CHO medium (GE Healthcare, Chicago, IL, USA), supplemented with 4 mM L-Glutamine (Lonza, Basel, Switzerland) and 10 g/L glucose (Roth, Karlsruhe, Germany). Cultivation was performed at 140 rpm (25 mm orbit) with 5 % CO₂ and 85 % humidity at 37 °C. Cells have been passaged every 3-4 days to a viable cell density (VCD) of 0.5 x 10⁶ cells/mL. VCD and viability have been determined via trypan blue exclusion using CEDEX XS (Roche Diagnostics, Mannheim, Germany). Transfection of CHO cells was performed using 15 µg vector using the NEON transfection kit (Thermo Fisher, Waltham, MA, USA). Cell lines stably expressing variants of the pEF-myc-cyto-mCMV-d2GFP (Addgene, Watertown, MA, USA) vector have been selected using 500 µg/mL G418 (Genaxxon, Ulm, Germany). To evaluate the response of CHO cells on undefined hypoxic cultivation conditions, CHO cells have been inoculated with a density of 0.5 x 10⁶ cells/mL and cultivated static with 5 % CO₂ and 85 % humidity at 37 °C. For cultivation at defined O₂ concentrations, CHO cells were inoculated in 1 L at a VCD of 0.3 x 10⁶ cells/mL using a 2 L stirred tank bioreactor (Sartorius, Göttingen, Germany). The bioreactors were controlling pH at 7.15, temperature at 37 °C, stirring speed at 100 rpm and O₂. The O₂ set point was adjusted every 24 h.

### Fed-batch fermentation

Fed-batch fermentation (fed-batch cell culture) was performed in a 2 L Biostat benchtop bioreactor (Sartorius Stedim Biotech GmbH, Goettingen, Germany). 3x10⁵ cells/mL were inoculated in 1 L SFM4CHO medium (GE Healthcare, Chicago, IL, USA), supplemented with 4 mM L-Glutamine (Lonza, Basel, Switzerland) and 10 g/L glucose (Roth, Karlsruhe, Germany). Fermentation was performed at 37 °C, pH 7.15, stirring speed at 100 rpm and an initial O₂ concentration of 40 %. During the process, the glucose concentration was maintained at 5 g/L and L-Glutamine at 2 mM. The main feed (1 L) consisted of 11.6 g HyClone Cell Boost 6 solved in 330 mL Millipore water (Cytiva, Marlborough, MA, USA), 20 g/L glucose and 660 mL SFM4CHO. The glutamine feed consisted of 100 mL 200 mM L-glutamine and 100 mL SFM4CHO. Viable cell density and viability was determined daily via trypan blue exclusion and after reaching the static cultivation phase, temperature was reduced to 34 °C and O₂ concentration in one fermenter to 1 %. Every day a sample was taken to determine SEAP concentration.

### Molecular biology

The starting plasmid 5HRE/GFP was a gift from Martin Brown & Thomas Foster (Addgene plasmid #46926) and has been described in Vordermark et al. 2001. Constructs were cloned using the following HRE sequences of the human vascular endothelial growth factor A (VEGF-A) and erythropoietin (EPO) genes (sequences are indicated in 5' -> 3' direction):
HRE sequence of the human VEGF-A gene:
   CCACAGTGCATACGTGGGCTCCAACAGGTCCTCTT (SEQ ID NO.: 1).
HRE sequence of the human EPO gene:
   GCCCTACGTGCTGTCTCACACAGCCTGTCTGAC (SEQ ID NO.: 28)

The oligonucleotides used are listed in Table 2 (Fw, forward; Rev, reverse; VEGF, VEGF-A). For cloning, oligonucleotides have been annealed by slowly reducing temperature from 95 °C to 25 °C, phosphorylated using T4 PNK (New England Biolabs, Ipswich, MA, USA) and ligated by T4 ligase (New England Biolabs, Ipswich, MA, USA). Randomly ligated double stranded oligonucleotides have been cloned into the pEF-myc-cyto-mCMV-d2GFP after removing the 5HREs by digesting with *XhoI* and *BglII* (both New England Biolabs, Ipswich, MA, USA).

**Table 2: Oligonucleotide sequences for molecular biology**

| Oligonucleotide | Sequence (indicated in 5' -> 3' direction) | Gene |
|---|---|---|
| Mock Fw | TCGAGACTAGTCCAGTGA (SEQ ID NO.: 29) | - |
| Mock Rev | GATCTCACTGGACTAGTC (SEQ ID NO.: 30) | - |
| VEGF-HRE-pair 1 Fw | TCGAGCCACAGTGCATACGTGGGCTCCAACAGG TCCTCTT (SEQ ID NO.: 31) | VEGF |
| VEGF-HRE-pair 1 Rev | CTCGACAAGAGGACCTGTTGGAGCCCACGTATG CACTGTGGC (SEQ ID NO.: 32) | VEGF |
| VEGF-HRE-pair 2 Fw | GTCGAGCCACAGTGCATACGTGGGCTCCAACAG GTCCTCTT (SEQ ID NO.: 33) | VEGF |
| VEGF-HRE-pair 2 Rev | CTCGACAAGAGGACCTGTTGGAGCCCACGTATG CACTGTGG (SEQ ID NO.: 34) | VEGF |
| VEGF-HRE-pair 3 Fw | GTCGAGCCACAGTGCATACGTGGGCTCCAACAG GTCCTCTTGTCGA (SEQ ID NO.: 35) | VEGF |
| VEGF-HRE-pair 3 Rev | GATCTCGACAAGAGGACCTGTTGGAGCCCACGT ATGCACTGTGG (SEQ ID NO.: 36) | VEGF |
| EPO-HRE-pair 1 Fw | TCGAGGCCCTACGTGCTGTCTCACACAGCCTGT CTGAC (SEQ ID NO.: 37) | EPO |
| EPO-HRE-pair 1 Rev | CTCGACGTCAGACAGGCTGTGTGAGACAGCACG TAGGGCC (SEQ ID NO.: 38) | EPO |
| EPO-HRE-pair 2 Fw | GTCGAGGCCCTACGTGCTGTCTCACACAGCCTG TCTGAC (SEQ ID NO.: 39) | EPO |
| EPO-HRE-pair 2 Rev | CTCGACGTCAGACAGGCTGTGTGAGACAGCACG TAGGGC (SEQ ID NO.: 40) | EPO |
| EPO-HRE-pair 3 Fw | GTCGAGGCCCTACGTGCTGTCTCACACAGCCTG TCTGACGTCGA (SEQ ID NO.: 41) | EPO |
| EPO-HRE-pair 3 Rev | GATCTCGACGTCAGACAGGCTGTGTGAGACAGC ACGTAGGGC (SEQ ID NO.: 42) | EPO |

To generate the vector for SEAP production, a DNA fragment comprising "5HRE-CMV-SEAP" was generated via gene synthesis and cloned into a suitable vector. 5HRE denotes five copies of the HRE sequence of the human VEGF-A gene, CMV denotes the complete CMV promoter and SEAP denotes the nucleic acid sequence encoding SEAP. Subsequently, the dihydrofolate reductase (DHFR) cassette was excised from the pOptiVEC-TOPO vector (Thermo Fisher Scientific, Darmstadt, Germany) and cloned into the "5HRE-CMV-SEAP"-comprising vector for selection purposes. The DHFR cassette was necessary for stable SEAP expression in CHO-DG44 (DHFR deficient) cells.

### RNA isolation

RNA isolation was performed using the miRNeasy Kit (Qiagen, Hilden, Germany) according to the manufacturer's protocol with 5 x 10⁶ cells. Purity and concentration of the isolated RNA was assessed using a Nanodrop 1000 spectrophotometer by absorbance at 260 nm (Thermo Fisher Scientific, Darmstadt, Germany).

### PCR and RT-PCR

After RNA isolation, cDNA was written using the Transcriptor High Fidelity cDNA Synthesis Kit (Roche Diagnostics, Mannheim, Germany) according to the manufacture's protocol. The transcription of HIF-1α, HIF-1β and VHL was demonstrated performing PCR on cDNA level with the following primers (sequences are indicated in 5' -> 3' direction; Fw denotes the forward primer; Rev denotes the reverse primer):

| | |
|---|---|
| HIF-1α Fw | TCCAGTTGCGCTCCTTTGAT (SEQ ID NO.: 43) |
| HIF-1α Rev | ATCCATTGATTGCCCCAGCA (SEQ ID NO.: 44) |
| HIF-1β Fw | TTGTCTCGTGTGAGACTGGC (SEQ ID NO.: 45) |
| HIP-1β Rev | CCAATGGCCACTAGGCAGAA (SEQ ID NO.: 46) |
| VHL Fw | GGTCATCTTCTGCAACCGCA (SEQ ID NO.: 47) |
| VHL Rev | AAGCTGGAATTCAGGACCACT (SEQ ID NO.: 48) |

Amplified DNA was separated via gel electrophoresis and detected using Fusion FX imager (Vilber Lourmat, Eberhardszell, Germany). Quantitative real-time PCR on d2GFP was performed using the following primers (sequences are indicated in 5' -> 3' direction; Fw denotes the forward primer; Rev denotes the reverse primer):

| | |
|---|---|
| d2GFP Fw | CTACCCCGACCACATGAAGC (SEQ ID NO.: 49) |
| d2GFP Rev | AAGTCGATGCCCTTCAGCTC (SEQ ID NO.: 50) |

with GAPDH as loading control:

| | |
|---|---|
| GAPDH Fw | GACTCTACCCATGGCAAGTTCA (SEQ ID NO.: 51) |
| GAPDH Rev | TCGCTCCTGGAAGATGGTGATG (SEQ ID NO.: 52) |

The measurement with SybrGreen Mastermix (Genaxxon, Ulm, Germany) was recorded using a Lightcycler480 (Roche Diagnostics, Mannheim, Germany).

### Flow cytometry analysis

GFP fluorescence of hypoxic and normoxic cultivated CHO cells was measured using MACSQuant Analyzer 10 without further staining (Miltenyi Biotec, Bergisch Gladbach, Germany). Resulting data was analyzed via the MACSQuantify software.

### Immunoblotting

Cells were lysed in RIPA buffer (1 % v/v NonidetTM P40, 0.5 % w/v sodium deoxycholate, ethylenediaminetetraacetic acid 1mM, all agents Carl Roth, Karlsruhe, Germany). Protein concentration was assed via BCA-assay. The sodium dodecyl sulfate polyacrylamide gel electrophoresis was performed using TruPAGE Precast 4-20 % gradient gels (Sigma-Aldich, St. Louis, MO, USA) with 30 µg protein per lane. Blotting of proteins was done on polyvinylidene fluoride membrane for 75 min with 60 mA and immunoblotting with the following antibodies was performed over night at 4 °C: anti-HIF1-alpha antibody (Bio-Techne, Minneapolis, MN, USA, #NB100-479SS, 1:1000); anti-VHL antibody (Santa Cruz Biotechnology, Dallas, TX, USA, #sc-135657 HRP, 1:200); anti-ARNT antibody (anti-HIP-1β antibody) (Santa Cruz Biotechnology, Dallas, TX, USA, #sc-17811, 1:200) and anti-β-actin antibody (Sigma-Aldrich, St. Lousis, MO, USA, #A5441-2ML, 1:1000). The secondary antibodies (anti-mouse IgG-HRP (Sigma Aldrich, St. Louis, MO, USA, #A4416; 1:5000); anti-rabbit IgG-HRP F(ab')₂ (Jackson ImmunoResearch, Cambridge, United Kingdom, #711-036-152, 1:50000)) were incubated for 1 h at room temperature. Detection was performed using Immobilon^{®} Western chemiluminescent HRP substrate (Millipore Corporation, Billerica, MA, USA) and Fusion FX imager (Vilber Lourmat, Eberhartszell, Germany).

### SEAP assay

Secreted embryonic alkaline phosphatase (SEAP) is a reporter widely used to study promoter activity or gene expression. To determine SEAP concentration in the SEAP reporter gene assay, chemiluminescent (Roche, Mannheim, Germany) was used according to the manufacturer's advice. Samples were heat inactivated at 65 °C for 30 min and have been diluted 1:50 to 1:300 depending on the SEAP concentration to fit to the linear area of the standard curve. Afterwards, 50 µL heat inactivated sample have been pipetted into a black non-binding flat bottom 96-well plate (Greiner Bio One, Frickenhausen, Germany) and incubated for 5 min with inactivation buffer and freshly prepared substrate was added. After 10 min incubation while gently shaking, luminescence was measured with 1 s integration time via SpectraMacs (Molecular Devices, San Jose, CA, USA).

### Statistical analysis

Statistical differences between data was determined using GraphPad Prism 6 software. ONE-WAY-ANOVA test with Bonferroni correction was conducted to calculate statistical differences of the mean between groups. All values of groups are shown as mean ± standard deviation (SD).

### Results

Before the scientific results are described in detail, it is referred to Figure 1, which shows the sequences of the HRE of the VEGF-A gene of various species in 5' -> 3' direction. The conserved binding site of HIF is underlined. It can be seen that the binding site is identically present in all sequences shown. It can further be seen that the sequences are highly conserved. The sequences differ between the species, if at all, only by slight modifications at a few specific positions that have been marked by vertical boxes for illustration purposes. The positions are nucleotide 1, nucleotide 19, nucleotide 21, nucleotide 23, nucleotide 31 and/or nucleotide 35.

The experimental results will now be described in detail.

### Presence and conservation of the hypoxia-responsive pathway in CHO cells

Besides hypoxia response elements (HREs) of hamster origin, HREs from other species such as human or mouse could serve as building blocks for the construction of hypoxia-responsive vectors to selectively enhance protein expression under hypoxic culture conditions in Chinese hamster ovary (CHO) cells. Among others, suitable elements to induce expression in CHO cells under hypoxic conditions could include HREs from the erythropoietin (EPO) and vascular endothelial growth factor A (VEGF-A) gene. To verify the potential binding of hamster proteins to human HREs in CHO cells, protein sequences of the key binding protein HIF-1α of human and hamster were aligned and analyzed for conservation of crucial domains (Figure 2A). For human HIF-1α three isoforms and for the hamster HIF-1α two isoforms displayed over 77 % conservation. Notably, critical areas like the DNA-binding domain, the HIP-1β heterodimerization domain and the nuclear localization signal were 100 % similar across all isoforms (Figure 2A). Especially the identical DNA-binding domain may allow the hamster HIF-1a to bind to human HREs introduced into CHO cells.

For the production of biopharmaceuticals, mainly CHO-K1 and CHO-DG44 cells are industrially used. To verify the suitability of these cell lines to introduce exogenous HREs, the expression of the essential hypoxia-related genes HIF-1α, HIP-1β and VHL was examined on RNA and protein level. For both cell lines HIF-1α and HIP-1β expression was detected using RT-PCR analysis (Figure 2B) as well as VHL, the antagonist to HIF-1α (Figure 2C). Finally, the protein expression of HIF-1α, HIP-1β and VHL was analysed via Western Blot (Figure 2D). While both VHL and HIP-1β were detected in both CHO-K1 and CHO-DG44 cultured under normoxic conditions, the instable HIF-1α could only be detected in CHO-DG44 cell lysates isolated from hypoxic cultivated cell cultures at around 100 kDa (Figure 2D). In summary, these data indicate the presence and conservation of key players of the hypoxia sensing pathway in CHO production cells which might therefore allow for cross species cell engineering.

### Vector development to enable hypoxia-inducible protein expression

To create and validate a hypoxia-inducible expression system, several vectors were designed carrying a minimal CMV (mCMV) promotor flanked upstream by HRE sequences and controlling the expression of the unstable d2GFP. HRE sequences were derived from hypoxia sensitive human genes EPO and VEGF-A and cloned as oligonucleotides with varying repetitions or without HREs to provide for a mock vector. A fragment of the vectors with HRE sequences is schematically shown in Figure 3A, in which 2-9 HREs denotes 2 to 9 copies of the HRE sequence of the human VEGF-A gene or the human EPO gene, mCMV denotes the minimal CMV promoter and d2GFP denotes the nucleic acid sequence encoding d2GFP. The HRE repetitions have a distance of six base pairs between adjacent HRE repeats. For all generated vectors (Table 3), stable cell pools for CHO-K1 and CHO-DG44 cell lines were subsequently generated.

**Table 3: Generated vector constructs for the analysis of HRE functionality**

| Vector name | Vector backbone | Origin of HRE sequence | Number of HRE repetitions |
|---|---|---|---|
| Mock | pEF-myc-cyto-mCMV-d2GFP | - | - |
| 2HRE-VEGF | pEF-myc-cyto-mCMV-d2GFP | VEGF-A | 2 |
| 5HRE-VEGF | pEF-myc-cyto-mCMV-d2GFP | VEGF-A | 5 |
| 8HRE-VEGF | pEF-myc-cyto-mCMV-d2GFP | VEGF-A | 8 |
| 3HRE-EPO | pEF-myc-cyto-mCMV-d2GFP | EPO | 3 |
| 4HRE-EPO | pEF-myc-cyto-mCMV-d2GFP | EPO | 4 |
| 9HRE-EPO | pEF-myc-cyto-mCMV-d2GFP | EPO | 9 |

### Induction of GFP expression under oxygen deprivation conditions

To verify the functionality of the resulting cell lines expressing GFP under the control of hypoxia-inducible elements, cell lines were initially cultivated in small scale batches under normal shaken and oxygen deprivation static conditions. Induced GFP expression was measured daily using flow cytometry. CHO-K1 cells expressing the mock vector showed no induction of GFP expression with a comparable minimal mean-fluorescence of around 0.3 - 0.5 under shaken and static cultivation between 24 h to 78 h (Figure 4A; in Figure 4, grey bars stand for shaken, while black bars represent static cultured pools). Similarly, CHO-K1 cells stably expressing the 2HRE-VEGF, 5HRE-VEGF or 8HRE-VEGF construct and cultivated under shaken normoxic conditions did not induce GFP expression displaying as well a GFP-mean fluorescence of around 0.3 - 0.5 (Figure 4A). In contrast, static cultured CHO-K1 cells showed a strongly increased GFP expression with mean fluorescence of 0.7 (2HRE-VEGF), 1.0 (5HRE-VEGF) and 2.0 (8HRE-VEGF) over time (Figure 4A). Comparable or even slightly pronounced induced expression results were obtained with CHO-DG44 cells, where mock expressing or shaken cultured cells showed no induced GFP expression with values between 0.3-0.6, whereas a strong increase in fluorescence intensity could be detected with statically cultivated 2HRE-VEGF (1.4), 5HRE-VEGF (3.0) and 8HRE-VEGF (2.4) CHO-DG44 cells (Figure 4C). When using CHO-K1 cells expressing hypoxia inducible constructs with 3, 4 or 9 HREs derived from the EPO gene, only a slight increase in GFP expression was observed for static (0.4 - 0.7) compared to mock or shaken (0.2 - 0.5) cultured cells (Figure 4B). CHO-DG44 cells on the other hand showed a remarkable induction of GFP expression (1.5 - 2.2) containing EPO-HRE constructs at static, hypoxia imitating conditions in comparison to the shaken cultured control cells (0.3 - 0.6) (Figure 4D). These data indicated that the VEGF-HRE constructs performed significantly better in both cell lines than the EPO-HRE counterparts. In addition, CHO-DG44 cells responded stronger to static and hypoxic cultivation with both VEGF- and EPO-HRE constructs than CHO-K1 cells. When using repetitive HRE sequences, a correlation between HRE number and expression response was observed, which was saturated between 5-8 HRE repetitions. Therefore, the 5HRE-VEGF construct was chosen for further analysis due to its strong induction of protein expression under oxygen limitation.

### Scale-up of batch fermentation under defined oxygen concentrations

After identifying the most hypoxia-responsive vector regarding type and number of HRE elements, the performance of the 5HRE-VEGF vector construct was further characterized in a scale up batch fermentation (batch cell culture) under defined oxygen concentrations. Both stable mock and 5HRE-VEGF expressing CHO-K1 and CHO-DG44 cell pools were cultivated as batch in a 2L-fermenter under normoxic conditions (60 % oxygen) for 156 hours. In addition, all cell lines were cultured in a batch fermentation where oxygen concentrations were switched to hypoxia starting after 12 hours and continuing in a stepwise manner (Figure 5A). For all fermentations, potential hypoxia-induced GFP fluorescence was recorded at regular intervals by flow cytometry. CHO-DG44 Mock and 5HRE-VEGF cells displayed identical mean fluorescence over the first 84 h of cultivation (Figure 5B). However, after a reduction to 5 % O₂ the CHO-DG44-5HRE-VEGF cells showed an induction of the GFP signal to about 0.6, which increased additionally significantly to 1.5 mean fluorescence at an oxygen concentration of 1 %. In contrast, the control cells showed continuously decreasing values from about 0.4 after 12 h to 0.15 after 156 h throughout the fermentation (Figure 5B). When comparing the mean fluorescence of normoxic and hypoxic CHO-DG44 Mock and 5HRE-VEGF cells, a significant nearly 10-fold induction of the GFP signal could be detected for CHO-DG44 5HRE-VEGF cells in comparison to the control cell lines (Figure 5C).

For CHO-K1 mock and CHO-K1-5HRE-VEGF cells, the cultivation under both normoxic and hypoxic conditions showed for the first 84 h hours of batch fermentation cultured a comparable pattern of the fluorescence signal of about 0.5 decreasing slightly but continuously to a value of about 0.25 (Figure 5D). After a decrease to 5% oxygen, CHO-K1-5HRE-VEGF cultured cells revealed a slight increase in mean fluorescence to 0.3, which further increased to 0.5 after a reduction to 1 % oxygen. Control cells decreased to about 0.15 at 5 % oxygen and 0.1 at 1 % oxygen (Figure 5E). When comparing the mean fluorescence of all cultured CHO-K1 cell lines after 140 h a significant stronger GFP expression could be observed in hypoxic cultured CHO-K1-5HRE-VEGF cell lines compared to Mock or normoxic 5HRE-VEGF control cell lines (Figure 5E). Interestingly, the hypoxic cultivation using oxygen concentrations of 5 % or 1 % had no significant negative effect on growth or viability of CHO-K1 cells (data not shown).

Taken together, these data point towards the successful establishment of a hypoxia-inducible vector system by using the 5HRE-VEGF constructs to drive protein expression under oxygen limitation.

The observed differences in the extent of induction of protein expression under hypoxic conditions between CHO-DG44 and CHO-K1 cells may be due to the fact that the basal expression of the HIF-1α protein is increased in CHO-DG44 cells compared to CHO-K1 cells (Figure 2D). In addition, the inventors found that the stabilization of the HIF-1α protein under hypoxic conditions is more effective in CHO-DG44 cells than in CHO-K1 cells (data not shown). This may explain the more effective induction of protein expression under hypoxic conditions observed in CHO-DG44 cells compared to CHO-K1 cells.

To further increase the induction of protein expression under hypoxic conditions, the cells may be further transformed so that (i) the cells overexpress HIF-1α, or (ii) the expression of the VHL protein is prevented or reduced. These measures may be particularly interesting for CHO-K1 cells in order to compensate for their HIF-1α deficiency.

### Increased recombinant protein expression exploiting hypoxia-responsive induction

To demonstrate the potential of HREs to improve recombinant protein expression, a stable secreted embryonic alkaline phosphatase (SEAP) expressing CHO-DG44 cell pool was generated with and without the introduction of 5 HREs originated from the VEGF-A gene upstream of the CMV promoter. A fragment of the SEAP expression vector having the 5 HREs is schematically shown in Figure 3B, in which 5HRE denotes five copies of the HRE sequence of the human VEGF-A gene, CMV denotes the complete CMV promoter and SEAP denotes the nucleic acid sequence encoding SEAP. SEAP and 5HRE-SEAP cells have been cultured under normal and static oxygen deprivation conditions. After 72 h SEAP titer was determined in all culture conditions and specific productivity calculated for comparison. Thereby, CHO-DG44-SEAP (SEAP) cells lost around 80 % of their specific productivity during hypoxia deprivation conditions in comparison to normally cultured cells (Figure 6A). In contrast, CHO-DG44-5HRE-SEAP (5HRE-SEAP) cells maintained over 50 % specific productivity during static cultivation and performed significantly better than cells lacking the HREs (Figure 6A). Additionally, the production capacity of SEAP and 5HRE-SEAP cells during very high cell densities was identified. In this context, both cell lines were inoculated with a normal (0.5 x 10⁶ cells/mL) and ultra-high (50 x 10⁶ cells/mL) cell density and a mock-perfusion batch was performed by replacing the medium daily. After 3 media replacements the specific productivities of SEAP and 5HRE-SEAP cells within 24 h were calculated revealing a slight decrease in specific productivity of ultra-high inoculated SEAP cells to around 80 % when compared to the normally inoculated control (Figure 6B). Strikingly, 5HRE-SEAP cells even increased their specific productivity during mock-perfusion conditions about 2.6-fold when compared to normally cultured 5HRE-SEAP cells leading to a significantly better performance during the mock-perfusion of cells carrying 5HREs of the gene VEGF-A (Figure 6B).

To finally validate the potential of HREs to improve the production capacity of CHO cells, a fed-batch fermentation of CHO-DG44-5HRE-SEAP cells was performed in a 2 L benchtop bioreactor. Cells were cultured under common conditions (37 °C, 40 % O₂) until reaching the static growth phase and then both a temperature shift to 34 °C alone and the combination of temperature shift and oxygen shift to 1 % was conducted. Although the same cell pool was cultured in both fermenters, the viable cell density (VCD) of one fermenter was slightly lower before the condition shift. However, after reducing temperature or temperature and oxygen concentration, VCD remained stable for both cultures (Figure 6C). Interestingly, a nearly identical viability for both culture conditions of >90 % was observed over 12 days, demonstrating the availability of CHO cells to be cultured under strong oxygen deprivation conditions (Figure 6D). By analyzing the SEAP titer of both fermenters a comparable SEAP concentration of around 13 µg/mL was monitored (Figure 6E) with a similar specific productivities of approximately 1.7 pg/cell/day over the first 6 days of cultivation (Figure 6F). Notably, after shifting temperature or temperature and oxygen concentration SEAP expression was induced in both fermenters, however, to a significant stronger extent in CHO cells benefiting from having adjusted the oxygen concentration to 1 % (Figures 6E and 6F). In this study, the hypoxia-inducible system led to a SEAP titer of around 460 µg/mL (Figure 6E) and a nearly 2-fold higher specific productivity than non-hypoxia-induced cells by reaching approximately 20 pg/cell/day SEAP expression (Figure 6F).

### References

Vordermark D, Shibata T, Brown JM. Green Fluorescent Protein is a Suitable Reporter of Tumor Hypoxia Despite an Oxygen Requirement for Chromophore Formation. Neoplasia. 2001;3: 527-534

## Claims

1. A method for producing a biomolecule by a cell cultured *in vitro,* the method comprising the steps of:
(a) propagating the cell in a cell culture, wherein the cell is transformed with a vector, wherein the vector comprises
(i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
(ii) a promoter that is active in the cell, wherein the promoter is operably linked to the HRE;
(iii) a nucleic acid sequence encoding the biomolecule, wherein the nucleic acid sequence is operably linked to the promoter;
and
(b) isolating the biomolecule from the cell culture.

2. The method according to claim 1, wherein the promoter is directly upstream of the nucleic acid sequence encoding the biomolecule.

3. The method according to claim 1 or claim 2, wherein the HRE is directly upstream of the promoter.

4. The method according to any one of claims 1 to 3, wherein the vector comprises at least two HREs arranged adjacent to one another, wherein the number of HREs preferably is between 2 and 20, further preferred between 2 and 10, further preferred between 2 and 8, most preferred 5.

5. The method according to any one of claims 1 to 4, wherein the cell is a Chinese hamster ovary (CHO) cell, a derivative of a CHO cell, a SL-29 cell, a duck embryo cell, a QT6 cell, a Vero cell, a NIH/3T3 cell, a BHK-21 cell, a Sp2/0-Ag14 cell, a BTI-Tn-5B1-4 cell, a hybridoma cell, a HeLa cell, a Human Embryonic Kidney 293 (HEK293) cell, a derivative of a HEK293 cell, a CAP cell or a CAP-T cell, wherein the derivative of the CHO cell preferably is a CHO-K1 cell, a CHO-EBNA cell, a CHO-EBNA GS cell, a CHO-DG44 cell, a CHO-DXB11 cell or a CHO-S cell, and wherein the derivative of the HEK293 cell preferably is a HEK293F cell or a HEK293S cell.

6. The method according to any one of claims 1 to 5, wherein the promoter is selected from the group consisting of cytomegalovirus (CMV) promoter, simian virus 40 early (SV40E) promoter, elongation factor 1 alpha (EF-1α) promoter, hEF1-HTLV promoter, T7 RNA polymerase and SP6 RNA polymerase (T7/SP6) promoter, ubiquitin C (UBC) promoter, U6 promoter, ferritin heavy chain (FerH) promoter, ferritin light chain (FerL) promoter, phosphoglycerate kinase (PGK) promoter, thyroxine-binding globulin (TBG) promoter, albumin (Alb) promoter and alpha-fetoprotein (AFP) promoter.

7. The method according to any one of claims 1 to 6, wherein the cell is further transformed with a nucleic acid sequence comprising a region encoding an RNA interference (RNAi) molecule that prevents or reduces expression of Von Hippel-Lindau protein, wherein the RNAi molecule is a small interfering RNA (siRNA), a short hairpin RNA (shRNA) or a microRNA (miRNA), and wherein the region encoding the RNAi molecule is operably linked to a promoter that is active in the cell.

8. The method according to claim 7, wherein the nucleic acid sequence comprising the region encoding the RNAi molecule is comprised by the vector.

9. The method according to claim 7 or 8, wherein the promoter to which the region encoding the RNAi molecule is operably linked, is operably linked to at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences.

10. The method according to any one of claims 1 to 9, wherein the cell is further transformed with a nucleic acid sequence encoding a HIF α subunit, wherein the nucleic acid sequence encoding the HIF α subunit is operably linked to a promoter that is active in the cell.

11. The method according to any one of claims 1 to 10, wherein the biomolecule is a recombinant peptide, a recombinant protein, a recombinant virus, a recombinant virus-like particle or a recombinant mRNA, wherein the recombinant protein preferably is an antibody.

12. The method according to any one of claims 1 to 11, wherein the cell culture is exposed to a hypoxic condition, wherein the hypoxic condition preferably is a level of oxygen from 0.1 % to 15 %, further preferred from 0.1 % to 10 %, further preferred from 1 % to 5 %, most preferred 1 %.

13. A cell for producing a biomolecule *in vitro,* wherein the cell is transformed with a vector, wherein the vector comprises
(i) at least one hypoxia-responsive element (HRE) consisting of a nucleic acid sequence selected from the group consisting of SEQ ID NO.: 1, SEQ ID NO.: 2, SEQ ID NO.: 3, SEQ ID NO.: 4 and functional variants of any one of these sequences;
(ii) a promoter that is active in the cell, wherein the promoter is operably linked to the HRE;
(iii) a nucleic acid sequence encoding the biomolecule, wherein the nucleic acid sequence is operably linked to the promoter.

14. The cell according to claim 13, wherein the cell is not a tumor cell.

15. The cell according to claim 13, wherein the cell is a Chinese hamster ovary (CHO) cell, a derivative of a CHO cell, a SL-29 cell, a duck embryo cell, a QT6 cell, a Vero cell, a NIH/3T3 cell, a BHK-21 cell, a Sp2/0-Ag14 cell, a BTI-Tn-5B1-4 cell, a hybridoma cell, a HeLa cell, a Human Embryonic Kidney 293 (HEK293) cell, a derivative of a HEK293 cell, a CAP cell or a CAP-T cell, wherein the derivative of the CHO cell preferably is a CHO-K1 cell, a CHO-EBNA cell, a CHO-EBNA GS cell, a CHO-DG44 cell, a CHO-DXB11 cell or a CHO-S cell, and wherein the derivative of the HEK293 cell preferably is a HEK293F cell or a HEK293S cell.
